# EUROPEAN PATENT SPECIFICATION

(11) **EP 2 214 000 B1**
(45) Date of publication and mention of the grant of the patent: **20.11.2013**
(21) Application number: 09151810.0
(22) Date of filing: 30.01.2009
(51) Int. Cl.: G01N 21/64, G01N 33/58

(54) **Use of FCCS for the analysis of interaction parameters in an in vivo-like environment**
Verwendung von FCCS zur Analyse von Wechselwirkungsparametern in einer in-vivo-artigen Umgebung
Utilisation de FCCS pour l'analyse de paramètres d'interaction dans un environnement de type in vivo

(43) Date of publication of application: 04.08.2010
(73) Proprietor: Intana Bioscience GmbH, 82152 Planegg-Martinsried (DE)
(72) Inventor: Hansen, Kerrin, 82205 Gilching (DE); Hannus, Stefan, 81373 Munich (DE); Becker, Frank, 82152 Planegg (DE)
(74) Representative: Bühler, Dirk

(56) References cited:
- WO-A-2008/019123
- US-A1- 2006 046 291
- US-A1- 2006 183 885
- THEWS E ET AL: "CROSS TALK FREE FLUORESCENCE CROSS CORRELATION SPECTROSCOPY IN LIVE CELLS" BIOPHYSICAL JOURNAL, NEW YORK, US, US, vol. 89, no. 3, 1 September 2005 (2005-09-01), pages 2069-2076, XP008056105 ISSN: 0006-3495
- KIM S A ET AL: "Two-photon cross-correlation analysis of intracellular reactions with variable stoichiometry" BIOPHYSICAL JOURNAL BIOPHYS. SOC USA, vol. 88, no. 6, June 2005 (2005-06), pages 4319-4336, XP002528709 ISSN: 0006-3495

### SEQUENCE LISTING

<110> Intana Bioscience
<120> Use of FCCS for the analysis of interaction parameters in an in vivo-like environment
<130> H 7417 / DB
<160> 4
<170> PatentIn version 3.3
<210> 1
   <211> 7495
   <212> DNA
   <213> Artificial
<220>
   <223> vector pGTOc-attR
<400> 1
   gacggatcgg gagatctccc gatcccctat ggtgcactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggag tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggaacc 720
   aaaatcaacg ggactttcca aaatgtcgta acaactccgc cccattgacg caaatgggcg 780
   gtaggcgtgt acggtgggag gtctatataa gcagagctct ccctatcagt gatagagatc 840
   tccctatcag tgatagagat cgtcgacgag ctcgtttagt gaaccgtcag atcgcctgga 900
   gacgccatcc acgctgtttt gacctccata gaagacaccg ggaccgatcc agcctccgga 960
   ctctagcgtt taaacgagct ccaccgcggt ggcggccgcc atggctagct ggagccaccc 1020
   gcagttcgaa aaagcggccg ccatggagag cgacgagagc ggcctgcccg ccatggagat 1080
   cgagtgccgc atcaccggca ccctgaacgg cgtggagttc gagctggtgg gcggcggaga 1140
   gggcaccccc gagcagggcc gcatgaccaa caagatgaag agcaccaaag gcgccctgac 1200
   cttcagcccc tacctgctga gccacgtgat gggctacggc ttctaccact tcggcaccta 1260
   ccccagcggc tacgagaacc ccttcctgca cgccatcaac aacggcggct acaccaacac 1320
   ccgcatcgag aagtacgagg acggcggcgt gctgcacgtg agcttcagct accgctacga 1380
   ggccggccgc gtgatcggcg acttcaaggt gatgggcacc ggcttccccg aggacagcgt 1440
   gatcttcacc gacaagatca tccgcagcaa cgccaccgtg gagcacctgc accccatggg 1500
   cgataacgat ctggatggca gcttcacccg caccttcagc ctgcgcgacg gcggctacta 1560
   cagctccgtg gtggacagcc acatgcactt caagagcgcc atccacccca gcatcctgca 1620
   gaacgggggc cccatgttcg ccttccgccg cgtggaggag gatcacagca acaccgagct 1680
   gggcatcgtg gagtaccagc acgccttcaa gaccccggat gcagatgccg gtgaagaaaa 1740
   gcccgggcgg atcatcacaa gtttgtacaa aaaagctgaa cgagaaacgt aaaatgatat 1800
   aaatatcaat atattaaatt agattttgca taaaaaacag actacataat actgtaaaac 1860
   acaacatatc cagtcatatt ggcggccgca ttaggcaccc caggctttac actttatgct 1920
   tccggctcgt ataatgtgtg gattttgagt taggatccgt cgagattttc aggagctaag 1980
   gaagctaaaa tggagaaaaa aatcactgga tataccaccg ttgatatatc ccaatggcat 2040
   cgtaaagaac attttgaggc atttcagtca gttgctcaat gtacctataa ccagaccgtt 2100
   cagctggata ttacggcctt tttaaagacc gtaaagaaaa ataagcacaa gttttatccg 2160
   gcctttattc acattcttgc ccgcctgatg aatgctcatc cggaattccg tatggcaatg 2220
   aaagacggtg agctggtgat atgggatagt gttcaccctt gttacaccgt tttccatgag 2280
   caaactgaaa cgttttcatc gctctggagt gaataccacg acgatttccg gcagtttcta 2340
   cacatatatt cgcaagatgt ggcgtgttac ggtgaaaacc tggcctattt ccctaaaggg 2400
   tttattgaga atatgttttt cgtctcagcc aatccctggg tgagtttcac cagttttgat 2460
   ttaaacgtgg ccaatatgga caacttcttc gcccccgttt tcaccatggg caaatattat 2520
   acgcaaggcg acaaggtgct gatgccgctg gcgattcagg ttcatcatgc cgtttgtgat 2580
   ggcttccatg tcggcagaat gcttaatgaa ttacaacagt actgcgatga gtggcagggc 2640
   ggggcgtaaa cgcgtggatc cggcttacta aaagccagat aacagtatgc gtatttgcgc 2700
   gctgattttt gcggtataag aatatatact gatatgtata cccgaagtat gtcaaaaaga 2760
   ggtatgctat gaagcagcgt attacagtga cagttgacag cgacagctat cagttgctca 2820
   aggcatatat gatgtcaata tctccggtct ggtaagcaca accatgcaga atgaagcccg 2880
   tcgtctgcgt gccgaacgct ggaaagcgga aaatcaggaa gggatggctg aggtcgcccg 2940
   gtttattgaa atgaacggct cttttgctga cgagaacagg ggctggtgaa atgcagttta 3000
   aggtttacac ctataaaaga gagagccgtt atcgtctgtt tgtggatgta cagagtgata 3060
   ttattgacac gcccgggcga cggatggtga tccccctggc cagtgcacgt ctgctgtcag 3120
   ataaagtctc ccgtgaactt tacccggtgg tgcatatcgg ggatgaaagc tggcgcatga 3180
   tgaccaccga tatggccagt gtgccggtct ccgttatcgg ggaagaagtg gctgatctca 3240
   gccaccgcga aaatgacatc aaaaacgcca ttaacctgat gttctgggga atataaatgt 3300
   caggctccct tatacacagc cagtctgcag gtcgaccata gtgactggat atgttgtgtt 3360
   ttacagcatt atgtagtctg ttttttatgc aaaatctaat ttaatatatt gatatttata 3420
   tcattttacg tttctcgttc agctttcttg tacaaagtgg tgattcgagg gggggcccgg 3480
   taccttgttt aaacccgctg atcagcctcg actgtgcctt ctagttgcca gccatctgtt 3540
   gtttgcccct cccccgtgcc ttccttgacc ctggaaggtg ccactcccac tgtcctttcc 3600
   taataaaatg aggaaattgc atcgcattgt ctgagtaggt gtcattctat tctggggggt 3660
   ggggtggggc aggacagcaa gggggaggat tgggaagaca atagcaggca tgctggggat 3720
   gcggtgggct ctatggcttc tgaggcggaa agaaccagct ggggctctag ggggtatccc 3780
   cacgcgccct gtagcggcgc attaagcgcg gcgggtgtgg tggttacgcg cagcgtgacc 3840
   gctacacttg ccagcgccct agcgcccgct cctttcgctt tcttcccttc ctttctcgcc 3900
   acgttcgccg gctttccccg tcaagctcta aatcgggggc tccctttagg gttccgattt 3960
   agtgctttac ggcacctcga ccccaaaaaa cttgattagg gtgatggttc acgtagtggg 4020
   ccatcgccct gatagacggt ttttcgccct ttgacgttgg agtccacgtt ctttaatagt 4080
   ggactcttgt tccaaactgg aacaacactc aaccctatct cggtctattc ttttgattta 4140
   taagggattt tgccgatttc ggcctattgg ttaaaaaatg agctgattta acaaaaattt 4200
   aacgcgaatt aattctgtgg aatgtgtgtc agttagggtg tggaaagtcc ccaggctccc 4260
   cagcaggcag aagtatgcaa agcatgcatc tcaattagtc agcaaccagg tgtggaaagt 4320
   ccccaggctc cccagcaggc agaagtatgc aaagcatgca tctcaattag tcagcaacca 4380
   tagtcccgcc cctaactccg cccatcccgc ccctaactcc gcccagttcc gcccattctc 4440
   cgccccatgg ctgactaatt ttttttattt atgcagaggc cgaggccgcc tctgcctctg 4500
   agctattcca gaagtagtga ggaggctttt ttggaggcct aggcttttgc aaaaagctcc 4560
   cgggagcttg tatatccatt ttcggatctg atcagcacgt gttgacaatt aatcatcggc 4620
   atagtatatc ggcatagtat aatacgacaa ggtgaggaac taaaccatgg ccaagttgac 4680
   cagtgccgtt ccggtgctca ccgcgcgcga cgtcgccgga gcggtcgagt tctggaccga 4740
   ccggctcggg ttctcccggg acttcgtgga ggacgacttc gccggtgtgg tccgggacga 4800
   cgtgaccctg ttcatcagcg cggtccagga ccaggtggtg ccggacaaca ccctggcctg 4860
   ggtgtgggtg cgcggcctgg acgagctgta cgccgagtgg tcggaggtcg tgtccacgaa 4920
   cttccgggac gcctccgggc cggccatgac cgagatcggc gagcagccgt gggggcggga 4980
   gttcgccctg cgcgacccgg ccggcaactg cgtgcacttc gtggccgagg agcaggactg 5040
   acacgtgcta cgagatttcg attccaccgc cgccttctat gaaaggttgg gcttcggaat 5100
   cgttttccgg gacgccggct ggatgatcct ccagcgcggg gatctcatgc tggagttctt 5160
   cgcccacccc aacttgttta ttgcagctta taatggttac aaataaagca atagcatcac 5220
   aaatttcaca aataaagcat ttttttcact gcattctagt tgtggtttgt ccaaactcat 5280
   caatgtatct tatcatgtct gtataccgtc gacctctagc tagagcttgg cgtaatcatg 5340
   gtcatagctg tttcctgtgt gaaattgtta tccgctcaca attccacaca acatacgagc 5400
   cggaagcata aagtgtaaag cctggggtgc ctaatgagtg agctaactca cattaattgc 5460
   gttgcgctca ctgcccgctt tccagtcggg aaacctgtcg tgccagctgc attaatgaat 5520
   cggccaacgc gcggggagag gcggtttgcg tattgggcgc tcttccgctt cctcgctcac 5580
   tgactcgctg cgctcggtcg ttcggctgcg gcgagcggta tcagctcact caaaggcggt 5640
   aatacggtta tccacagaat caggggataa cgcaggaaag aacatgtgag caaaaggcca 5700
   gcaaaaggcc aggaaccgta aaaaggccgc gttgctggcg tttttccata ggctccgccc 5760
   ccctgacgag catcacaaaa atcgacgctc aagtcagagg tggcgaaacc cgacaggact 5820
   ataaagatac caggcgtttc cccctggaag ctccctcgtg cgctctcctg ttccgaccct 5880
   gccgcttacc ggatacctgt ccgcctttct cccttcggga agcgtggcgc tttctcatag 5940
   ctcacgctgt aggtatctca gttcggtgta ggtcgttcgc tccaagctgg gctgtgtgca 6000
   cgaacccccc gttcagcccg accgctgcgc cttatccggt aactatcgtc ttgagtccaa 6060
   cccggtaaga cacgacttat cgccactggc agcagccact ggtaacagga ttagcagagc 6120
   gaggtatgta ggcggtgcta cagagttctt gaagtggtgg cctaactacg gctacactag 6180
   aagaacagta tttggtatct gcgctctgct gaagccagtt accttcggaa aaagagttgg 6240
   tagctcttga tccggcaaac aaaccaccgc tggtagcggt ttttttgttt gcaagcagca 6300
   gattacgcgc agaaaaaaag gatctcaaga agatcctttg atcttttcta cggggtctga 6360
   cgctcagtgg aacgaaaact cacgttaagg gattttggtc atgagattat caaaaaggat 6420
   cttcacctag atccttttaa attaaaaatg aagttttaaa tcaatctaaa gtatatatga 6480
   gtaaacttgg tctgacagtt accaatgctt aatcagtgag gcacctatct cagcgatctg 6540
   tctatttcgt tcatccatag ttgcctgact ccccgtcgtg tagataacta cgatacggga 6600
   gggcttacca tctggcccca gtgctgcaat gataccgcga gacccacgct caccggctcc 6660
   agatttatca gcaataaacc agccagccgg aagggccgag cgcagaagtg gtcctgcaac 6720
   tttatccgcc tccatccagt ctattaattg ttgccgggaa gctagagtaa gtagttcgcc 6780
   agttaatagt ttgcgcaacg ttgttgccat tgctacaggc atcgtggtgt cacgctcgtc 6840
   gtttggtatg gcttcattca gctccggttc ccaacgatca aggcgagtta catgatcccc 6900
   catgttgtgc aaaaaagcgg ttagctcctt cggtcctccg atcgttgtca gaagtaagtt 6960
   ggccgcagtg ttatcactca tggttatggc agcactgcat aattctctta ctgtcatgcc 7020
   atccgtaaga tgcttttctg tgactggtga gtactcaacc aagtcattct gagaatagtg 7080
   tatgcggcga ccgagttgct cttgcccggc gtcaatacgg gataataccg cgccacatag 7140
   cagaacttta aaagtgctca tcattggaaa acgttcttcg gggcgaaaac tctcaaggat 7200
   cttaccgctg ttgagatcca gttcgatgta acccactcgt gcacccaact gatcttcagc 7260
   atcttttact ttcaccagcg tttctgggtg agcaaaaaca ggaaggcaaa atgccgcaaa 7320
   aaagggaata agggcgacac ggaaatgttg aatactcata ctcttccttt ttcaatatta 7380
   ttgaagcatt tatcagggtt attgtctcat gagcggatac atatttgaat gtatttagaa 7440
   aaataaacaa ataggggttc cgcgcacatt tccccgaaaa gtgccacctg acgtc 7495
<210> 2
   <211> 1002
   <212> DNA
   <213> Homo sapiens
<220>
   <221> misc_feature
   <223> coding sequence of the catalytic domain of bRAF
<400> 2
   gaagacagga atcgaatgaa aacacttggt agacgggact cgagtgatga ttgggagatt      60
   cctgatgggc agattacagt gggacaaaga attggatctg gatcatttgg aacagtctac     120
   aagggaaagt ggcatggtga tgtggcagtg aaaatgttga atgtgacagc acctacacct     180
   cagcagttac aagccttcaa aaatgaagta ggagtactca ggaaaacacg acatgtgaat     240
   atcctactct tcatgggcta ttccacaaag ccacaactgg ctattgttac ccagtggtgt     300
   gagggctcca gcttgtatca ccatctccat atcattgaga ccaaatttga gatgatcaaa     360
   cttatagata ttgcacgaca gactgcacag ggcatggatt acttacacgc caagtcaatc     420
   atccacagag acctcaagag taataatata tttcttcatg aagacctcac agtaaaaata     480
   ggtgattttg gtctagctac agtgaaatct cgatggagtg ggtcccatca gtttgaacag     540
   ttgtctggat ccattttgtg gatggcacca gaagtcatca gaatgcaaga taaaaatcca     600
   tacagctttc agtcagatgt atatgcattt ggaattgttc tgtatgaatt gatgactgga     660
   cagttacctt attcaaacat caacaacagg gaccagataa tttttatggt gggacgagga     720
   tacctgtctc cagatctcag taaggtacgg agtaactgtc caaaagccat gaagagatta     780
   atggcagagt gcctcaaaaa gaaaagagat gagagaccac tctttcccca aattctcgcc     840
   tctattgagc tgctggcccg ctcattgcca aaaattcacc gcagtgcatc agaaccctcc     900
   ttgaatcggg ctggtttcca aacagaggat tttagtctat atgcttgtgc ttctccaaaa     960
   acacccatcc aggcaggggg atatggtgcg tttcctgtcc ac                       1002
<210> 3
   <211> 120
   <212> DNA
   <213> Artificial
<220>
   <223> part of vector sequence pGTO-bRAF (around 5' att site)
<400> 3
   gatgccggtg aagaaaagcc cgggcggatc atcacaagtt tgtacaaaaa agcaggctgt      60
   cgaccatcag ccctcaagca gtggtatcaa cgcagagtac gcggggaaga caggaatcga     120
<210> 4
   <211> 72
   <212> DNA
   <213> Artificial
<220>
   <223> part of vector sequence pGTO-bRAF (around 3' att site)
<400> 4
   gcgtttcctg tccactgaat cgtaggcggc cgcccagctt tcttgtacaa agtggtgatt    60
   cgaggggggg cc                                                        72

## Description

### Field of the Invention

The present invention relates to the pharmaceutical field, in particular to the field of drug discovery where *inter alia* interaction parameters between two molecules are of major interest.

With respect to such parameters, the present invention relates *inter alia* to the determination of interaction parameters between a target of pharmaceutical interest and a small molecule compound having potential impact on said target by employing fluorescence cross correlation spectroscopy (FCCS) in an *in vivo*-like environment.

In general, the present invention which is defined in claim 1 relates to a method of determining interaction parameters of at least two analytes comprising *inter alia* the provision of an FCCS device, the provision of a lysate of cells and the determination of interaction parameters of said analytes in said lysate by FCCS.

Furthermore, the present invention defined in claims 2 and 3 relates to a method of determining interaction parameters for at least one competitive agent influencing the interaction of at least two analytes comprising *inter alia* the provision of an FCCS device, the provision of a lysate of cells and the at least one competitive agent and the determination of interaction parameters of said analytes in said lysate by FCCS.

### Background of the invention

Over the past years, research has led to an overwhelming depth of information about causes for diseases on a molecular level and about correlations on a molecular level. Thus, it is possible today to assign a large number of diseases to main cellular factors (e.g. proteins) which seem to be disease causing (e.g. constitutively active enzymes).

A very promising approach for curing said diseases is the specific targeting of such main cellular factors (also termed targets) by appropriate molecules (e.g. small molecule compounds, also termed compounds) with the goal of interfering with the disease-causing molecular event (e.g. by inhibiting a constitutively active enzyme).

One example in this respect represents the finding that active bRAF kinase signalling in mammalian cells is one major factor for the development of cancer. bRAF kinase thus represents one of the targets as outlined above. Therefore, the goal is to specifically inhibit bRAF signalling by corresponding inhibitors, and one prominent inhibitor, namely compound BAY43-9006, could be identified using appropriate screening strategies followed by optimization procedures.

For example, two- or three-hybrid approaches and approaches using the analysis of complexes by mass spectrometry are performed as main initial screening methods in pharmaceutical industry today in order to identify candidate compounds for a certain target.

Subsequently, the binding of a candidate compound to a target needs to be confirmed and analyzed by at least one method differing from the screening method.

Furthermore, almost all of said initially found compounds need to be optimized before they may be used as medicaments as they may e.g. exhibit weak binding activity or unfavourable chemical characteristics for active agents in medicaments. Interaction parameter such as Kd-, kon- and koff-values and the like represent important information for the screening, characterization and optimization procedures as outlined above.

Said interaction parameters are mostly determined in *in vitro* assays, such as e.g. ELISA-assays.

An experimental setup *in vitro* is, however, afflicted with disadvantages since the interaction of a target and a compound will be in a different environment, namely a cellular environment, when the compound is eventually applied in the form of an active agent in a medicament.

Thus, information on interaction parameters *in vivo* is of major interest. Depending on the interaction analyzed, methods for determining said parameters *in vivo* are either not at all available or to a limited amount only.

THEWS E ET AL: "CROSS TALK FREE FLUORESCENCE CROSS CORRELATION SPECTROSCOPY IN LIVE CELLS", BIOPHYSICAL JOURNAL, NEW YORK, US, vol. 89, no. 3, 1 September 2005 (2005-09-01), pages 2069-2076, ISSN: 0006-3495 describes the use of fluorescence correlation spectroscopy for determining interaction parameters of two proteins in an *in* vivo-like environment.

Thus, there is the need for an experimental system which is capable of analyzing interaction parameters of a target and a compound in an at least *in vivo*-like environment without necessarily requiring further information on e.g. initial concentrations of said interaction partners in the system.

### Objects and summary of the invention

It is an objective of the present invention to provide a method that can be used for the determination of interaction parameters of at least two analytes in an *in vivo*-like environment.

Furthermore, it is an objective of the present invention to provide a method that can be used for the determination of interaction parameters for at least one competitive agent influencing the interaction of at least two analytes in an *in vivo*-like environment.

These and other objectives of the present invention, as they will become apparent from the ensuing description, are solved by the subject matter of the independent claims. The dependent claims relate to some of the preferred embodiments of the invention.

The analytes mentioned in step b), step c) and step f) of claims 1-3 may also be designated as a first analyte (step b)) and a second analyte (step c) or step f)).

The competitive agent used in step c) or f) of claims 3 and 2 does not necessarily need to be fluorescently labelled. It is preferred that the competitive agent is not labelled.

One may either establish an interaction between at least two analytes first and then add the at least one competitive agent or establish an interaction between at least one analyte and at least one competitive agent first and then add the at least one further analyte.

No information on the initial concentrations of said at least two analytes and/or said at least one competitive agent is required.

In further preferred embodiments of the methods of the present invention, the cells cultured outside the human or animal body as mentioned in step b) are mammalian cells selected from the group of cells comprising HEK 293, HEK 293 T, HeLa and HUVEC cells.

Preferably, said cells are HEK 293 cells, optionally T-REX^{™} HEK 293 cells from Invitrogen, Germany.

Furthermore, according to preferred embodiments of the methods of the present invention, the cells mentioned in step b) are lysed in order to provide a lysate as sample wherein said lysis is in preferred embodiments performed according to the following protocol: After collecting and washing of cells, the cells are centrifuged and lysed by adding hypotonic buffer as well as douncing with a glass douncer using a tight-fitting pestle. After restoring physiological salt concentrations, the lysate is transferred into an ultracentrifuge tube and spun, wherein the resulting supernatant represents the sample. It is preferred that said hypotonic buffer does not comprise any detergent at all. However, in other equally preferred embodiments, a detergent may be used wherein the concentration of said detergent is below the critical micellular concentration (CMC). For all lysis protocols it is preferred that they are carried out on ice with buffers and solutions used at a temperature of 4°C.

In an especially preferred embodiment, said fluorescent label is an autofluorescent protein selected from the group of autofluorescent proteins comprising GFP, YFP, CFP and RFP with GFP being particularly preferred.

Furthermore, in a preferred embodiment of the invention, said fluorescent label is a fluorescent dye selected from the group of fluorescent dyes comprising Cy3, Cy5 and Alexa-dyes as well as derivatives thereof. In a particularly preferred embodiment, said fluorescent label is Cy5.

In further preferred embodiments according to the methods of the present invention, the fluorescent labels which are e.g. attached to the at least first and/or second analyte are different fluorescent labels regarding their fluorescent properties when used together in a sample. Thus, at least two different fluorescent labels are comprised in one sample. The different fluorescent labels are generally chosen such that their spectroscopic properties do not substantially overlap meaning that the labels emit at wavelengths sufficiently distinct from each other such that they can be detected with an FCCS device.

It is thus for example preferred to use GFP and Cy5, Alexa-488 and RFP, or YFP and CFP together with optionally further different fluorescent labels in combination as fluorescent labels of the at least two analytes in a sample. According to the preferred embodiments as mentioned above, it is particularly preferred to use GFP and Cy5 in combination when interaction parameters of two analytes or the interaction parameters for a competitive agent influencing the interaction of two analytes are determined.

According to the present invention defined in claims 2 and 3, said competitive agent is a small molecule compound. In this case, one of said at least two analytes is also a small molecule compound, whereas the second analyte is a fusion protein comprised of a target protein and an autofluorescent protein.

In certain aspects of the invention, it is preferred that the interaction parameters determined comprise affinity parameters and kinetic parameters.

Said affinity parameters determined according to the methods of the present invention comprise preferably Kd-, Ki and IC50-values.

Said kinetic parameters determined according to the methods of the present invention comprise preferably kon-, koff- and kobs-values.

### Description of the figures

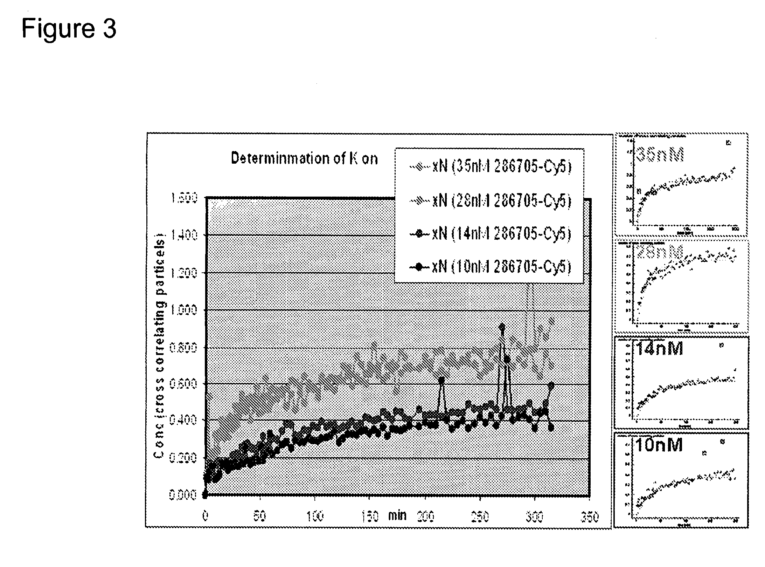

Figure 1:
   **Plasmid map of pGTOc-attR.** Main features of vector pGTOc-attR used for expression of the GFP-target fusion proteins are depicted. The target sequence is inserted via Gateway^{™} cloning procedures using the att-sites.
Figure 2:
   **Titration of labelled compound vs. lysate comprising a GFP-target fusion.** Cy5-labelled PD-173956 was titrated in a concentration range between 1 nM and 100 nM to aliquots of a lysate comprising GFP-bRAF (log-scale on x-axis). Kd-values can be deduced within the linear range of the curve.
Figure 3:
   **Determination of the kinetic parameters kon and koff via kobs (1).** Cy5-labelled Bay43-9006 (also termed and here depicted as "286705") was added in four different concentrations as shown in the figure legend (10, 14, 28 and 35 nM) to lysate comprising GFP-bRAF and incubated. Data points were taken every two minutes over a total incubation time of 320 minutes. The concentration of cross correlating particles is depicted vs. the incubation time and an overlay of the recorded binding curves is shown in the left panel with individual curves on the right comprising data points and fitted graphs.
Figure 4:
   **Determination of the kinetic parameters kon and koff via kobs (2).** The fitted data depicted in Figure 3 were exported to EXCEL® fit and, using said software, fitted again in order to determine Kon- and koff-values using a linear regression algorithm. The data correspond to the kinetics of the interaction of Bay43-9006-Cy5 and GFP-bRAF as described above.
Figure 5:
   **Effect of a competitive agent on an established interaction.** First, the interaction between PD-173956-Cy5 and GFP-bRAF was established. An unlabelled competitive agent was then added to the complex (in A: Bay43-9006; in B: PD-173956) in different concentrations (as shown on the x-axis of the graphs) and samples were incubated for 5 hours to reach steady state. Following the incubation, FCCS measurements were performed. The determined cross correlation amplitude values were then blotted vs. the concentrations of the competitive agents and fitted. IC₅₀ values can be then be determined from the graphs (in both cases about 20 nM).
Figure 6:
   **Determination of kinetic parameters for a competitive reaction**. An interaction between GFP-bRAF and PD-173956-Cy5 was established. Unlabelled Bay43-9006 in different concentration (62, 125, 250 and 500 nM) was then added to aliquots of the established interaction and the reactions were followed by determining cross-correlating particles every 3 minutes over an incubation time of 440 minutes. Top: concentration of cross correlating particles vs. the incubation time at the different concentrations with fitted graphs; Bottom: Data were exported and fitted by EXCEL® fit in order to determine Kon- and koff-values using a linear regression algorithm wherein the concentration of the competitor is blotted vs. the kobs-values.
Figure 7:
   **Direct determination of Koff in a competitive reaction**. An interaction between GFP-bRAF and unlabelled Bay43-9006 was established by incubating the lysate and the compound overnight. PD-173956-Cy5 was then added in excess (300 nM) and the reaction was followed by determining cross-correlating particles every 3 minutes over a period of 440 minutes.

### Detailed description of the invention

The inventors have found that it is possible to determine interaction parameters of at least two analytes in an *in vivo*-like environment using FCCS, as defined in claim 1.

Furthermore, the inventors have found that said method can also be employed to determine interaction parameters for at least one competitive agent influencing the interaction of at least two analytes, as defined in claims 2 and 3.

The use of FCCS according to the invention has several advantages over other methods used to confirm, characterize and/or screen for interactions (such as e.g. pull-down, immunoprecipitation or enzymatic assays). FCCS as used according to the invention displays a high sensitivity, a broad dynamic range (e.g. Kd-values over a range of 0,1 nM to 5 µM may be determined), is highly economic due to the low input of substances (such as e.g. the amount of the fluorescent dye-labelled small molecule compounds) and the small volume of the sample, fast, reliable and displays a wide variety of potential applications. Most importantly, FCCS may be used according to the invention in an *in vivo*-like environment since cellular lysates are used.

While describing in detail exemplary embodiments of the present invention, definitions important for understanding the present invention are provided.

In the context of the present invention, the term "about" and "approximately" denote an interval of accuracy that a person skilled in the art will understand to still ensure the technical effect of the feature in question. The term typically indicates a deviation from the indicated numerical value of ±10% and preferably ±5%.

As has been set out above, the present invention relates in one aspect to a method of determining interaction parameters of two analytes as defined in claim 1.

Said aspect of the invention may preferably be used when confirming the interaction between e.g. a target and a compound wherein said interaction has been found in an initial screen. The interaction may then be confirmed in an *in vivo*-like environment by using the method of the present invention. However, the analysis may also lead to the result that the two analytes do not interact under conditions used herein, i.e. in contradiction to previous results gained by other methods, e.g. by *in vitro* methods.

However, said aspect of the invention may also be used to further characterize said interaction by determining additional interaction parameters such as kinetic parameters in an *in vivo*-like environment.

Using the method of the present invention, it is furthermore possible to gain additional information (apart from interaction parameters of at least two analytes), such as information on how specific the interaction of two analytes is in an *in vivo-*like environment. Thus, it is possible to quantitatively determine "off-target" effects (i.e. interactions between an analyte and other factors present in the lysate), which may be disadvantageous in later applications.

Furthermore, said aspect of the invention may also be used in order to screen for analytes or compounds exhibiting binding activities towards a target. In this embodiment of the invention, a target may be present as fusion protein in a lysate and different compounds of a library of compounds may be added to aliquots of the lysate followed by the determination of interaction parameters of the different compounds and the target. Compounds exhibiting strong binding affinities towards the target may thus be identified in an *in vivo*-like environment.

Said aspect of the invention may also be used in order to screen for targets exhibiting binding activities towards a compound. In this embodiment of the invention, different targets (in certain embodiments a library of targets or different mutant versions of the target representing e.g. versions of clinical relevance deriving from different SNPs) may be expressed as fusion proteins in cells followed by the lysis of said cells. Thus, different lysates, each containing a specific fusion protein may be obtained. To said different lysates, an identical compound may then be added followed by the determination of interaction parameters of the different fusion proteins and the compound. Targets exhibiting strong binding affinities towards the compound may thus be identified in an *in vivo*-like environment.

In summary, the method as described above represents a very easy way of confirming and characterizing interaction parameters, obtaining information on off-target effects as well as screening for interacting analytes in an *in vivo*-like environment. Said method may preferably be used in compound and/or target identification, characterization and screening processes.

As has also been set out above, the present invention relates in another aspect to a method of determining interaction parameters for at least one competitive agent influencing the interaction of at least two analytes as defined in claims 2 and 3.

In this aspect of the invention, the main goal may be formulated as characterizing and screening for potentially optimized compounds compared to a compound found in an initial screen (e.g. to further analyze the structure-action-relationship [SAR] of modified compounds). For example, a higher binding affinity may be advantageous or there may be the need to optimize the initial compound regarding its chemical nature in order to fulfil certain required characteristics as active agent in a medicament (such as e.g. the introduction of hydrophobic parts to allow for membrane crossing).

Said aspect of the invention may be used in order to determine whether such a modified compound exhibits advanced characteristics compared to the initial compound. Furthermore, it is possible to screen a large number of derivatives of an initial compound or a library of compounds based on an initial compound in a very easy and reliable way. Said derivatives or library members may be used in an unmodified, i.e. unlabelled, way, which is very cost-effective since the interaction to be competed with is established using two labelled analytes. Also, several of said derivatives may be combined.

In summary, said second aspect of to the invention represents an easy way of either determining whether an alleged optimized compound really displays advanced characteristics over an existing compound or of screening for potential optimized compounds. Thus, said method may preferably be used in compound optimization processes.

General terms as used in the description of the present invention will now be explained and defined in the following section of the description.

The term "interaction parameter" is used herein as known to the skilled person. Said term can be described as defining the interaction between at least two analytes or interaction partners, i.e. the binding affinity between two molecules, and/or as defining the reaction rates of the reaction between said two molecules, i.e. the kinetic of the reaction (e.g. the association and dissociation reaction of the overall reaction between said two molecules). All of the interaction parameters as explained in the following are also used as known to the skilled person.

Typically, the dissociation constant Kd describes the affinity between two analytes, e.g. between a compound (C) and a protein (P). The reaction between C and P can be expressed as C + P = CP with C and P being unbound fractions and CP being a complex of C and P. The dissociation constant Kd is defined by Kd = [C] x [P] / [CP] (with [X] being the concentration of X in the reaction). Kd is expressed in molar units M and corresponds to the concentration of C, at which the concentration of P bound to C equals the concentration of P. The smaller the Kd-value, the higher the affinity between C and P.

If a competitor (K) for C is added to the reaction above, the Ki-value corresponds to the Kd-value of the interaction between competitor K and P. However, in order to determine said Ki-value, the IC₅₀-value (see below) needs to be determined. In a second step, the Ki-value may then be determined by applying the Cheng-Prusoff Equation Ki = IC₅₀ / (1 + ([K] / Kd)). As above, the smaller the Ki-value, the higher the affinity between K and P.

With the above definitions and components, the IC₅₀-value can be explained as measuring the effectiveness of competitor K in inhibiting the binding reaction between C and P. The IC₅₀-value is expressed in molar units M and corresponds to the half maximal (50) inhibitory concentration (IC) of K for inhibiting binding of C to P. In order to determine the IC₅₀-value, it can be necessary to apply a dilution series with different concentration of K.

For a reaction as outlined above, there is also a reaction rate of association of P and C in order to form PC and a reaction rate of dissociation of PC to P and C. The reaction rate of association may be referred to as Kon, whereas the reaction rate of dissociation may be referred to as Koff. The observed reaction rate of a reaction is, however, also influenced by the concentration of the complex PC, [PC]. For the kinetic of a reaction, the observed reaction rate may thus be expressed as follows: Kobs = Kon x [PS] + koff. Once the reaction is in equilibrium with a constant concentration of PS, the observed reaction rate Kobs does not change any more. Furthermore, the Kd value as discussed above may be expressed as Kd = kon / koff.

The term "analytes" as used herein refers to two molecules as defined in the claims, which may interact and referred to as interaction partners in the following. In this case, parameters of said interaction may be determined according to the invention. However, if said two analytes do not interact in the *in vivo* like environment used in the present invention, the overall result will be that said two analytes do not interact under conditions used herein. Such a result may be of particular relevance if an interaction between two analytes has been established in previous assays employing purified *in vitro* conditions only.

In general, the analytes may optionally be modified. Modifications comprise sugar moieties, phosphorylations, acetylations and the like, ubiquitylation, SUMOylation and further protein modifiers, lipids as well as linker structures introduced or the like. According to the invention one analyte is a protein (which represents a target of pharmaceutical interest) and the other analyte is a small molecule compound (which may inhibit the target of pharmaceutical interest). If a competitive agent is used in certain embodiments of the invention, it is a small molecular compound as defined in claims 2 and 3.

The term "small molecule compound" is used as common in the art. It thus does not include e.g. proteins, peptides and nucleic acids.

The term "peptide" refers to a stretch of typically 3 to 30, preferably of 4 to 25, more preferably of 5 to 20 amino acids linked by peptide bonds.

The analytes are selected from proteins and small molecule compounds, as defined in the claims.

The term "*in vivo*-like environment" as used herein describes the condition of the sample used for determining interaction. Said sample does not only consist of an appropriate buffer and the two purified analytes (as e.g. in *in vitro* systems), but is, due to the lysis protocol as outlined below, rather comprised of the main cellular factors (and thus *in vivo* like). Such lysates may typically not comprise artificial lipid vesicles or micelles, which may incorporate or shield analytes resulting in artefacts or no data at all.

In the following, all steps of the method of the invention comprising an FCCS device will be described in more detail with references to the general mode of action of an FCCS device. According to the present invention, an FCCS device comprising a loading zone for a sample needs to be provided. Furthermore, said loading zone of the device is loaded with a sample and interaction parameters of at least two analytes present in said sample are determined.

First, an overview of the general setup of an FCCS device and the mode of operation will be given. Said overview is restricted to an FCCS device using two colour fluorescence. However, this should not be interpreted as limiting, since the system as described below will, in principle, also work with three or more colour fluorescence.

First of all it should be mentioned that any FCCS device known to the skilled person may be used for the method of the present invention. Today, said devices are commercially available and examples for commercially available FCCS devices which may be used in accordance with the present invention are the "Zeiss® ConfoCor® 2" and the "Zeiss® ConfoCor® 3" by Zeiss, Jena, Germany.

An FCCS device is typically comprised of a laser unit, a microscope, a loading zone for a sample, a confocally arranged optical setup and a detector unit. For further details of this setup and a detailed introduction into the mode of action of an FCCS device, reference is made to Weisshart et al., Curr. Pharm. Biotechnology, 2004, 5, 135-154.

The system as described below can be used to determine the interaction parameters of at least two analytes, such as e.g. a fluorescently labelled protein and a fluorescently labelled compound in an *in vivo* like environment. However, in the following introductory section, reference is made to two different fluorescent molecules only representing said labelled analytes.

In a very simplified way, the method of operation of said FCCS device may be explained as follows. In the first step, a sample comprising two said two different fluorescent molecules is loaded onto the loading zone of the FCCS device.

Typically, before loading, the sample is transferred into a small transparent well of a multiple well plate, preferably a 384 well plate. However, any other multiple well plate may also be used, such as a 16, 32, 64, 128 or 256 well plate. The sample may also be in a single chamber or well without the need of transferring the sample or in any other appropriate device which can be used together with the loading zone of the FCCS device.

The sample is typically a liquid sample of a small volume. Thus, a liquid sample such as e.g. a cell lysate may be pipetted into a small transparent well as set out above with the liquid sample having a volume of between about 1 µl and about 1 ml, preferably between about 5 µl and about 500 µl, more preferably between about 20 µl and 80 µl with about 40 µl being the most preferred volume.

In the second step, the objective lense of the microscope of the FCCS device needs to be chosen and prepared. Any of the objective lenses of the microscope may be used. However, it is preferred to use in case of the Zeiss® ConfoCor® 2 the objective "40x / 1.2 W Korr" which allows, together with the optical setup of the FCCS device, the detection of fluorescence in a detection volume of about 0,25 fl. However, any other objective and detection volume may also be used in accordance with to the present invention and dependent on the FCCS device used. Preparation of the objective is typically done by adding a drop of water onto the lens as immersion-medium.

In the third step, the single or multiple well plate, e.g. the 384 well plate, comprising the at least one liquid sample of a small volume in at least one wells, is loaded onto the loading zone of the FCCS device, which is located onto the objective of the microscope.

Thus, after the FCCS device has been loaded with e.g. a transparent well comprising the liquid sample, a microscopic small volume (e.g. 0,25 fl) within said small total volume of the sample (e.g. about 40 µl) is in the subsequent steps used as excitation and detection volume in order to follow single fluorescent molecules diffusing into and out of said microscopic small volume.

In order to excite a fluorescent molecule within said microscopic small volume, light of a specific wavelength needs to be employed. In the FCCS setup, two strongly focussed excitation laser beams are used and emit light of two specific wavelengths into the microscopic small volume as defined above. Any appropriate combination of two laser beams may be used in accordance with the fluorescent molecules used as labels. However, it is preferred to use a laser beam emitting light of a wavelength of 488 nm in order to excite GFP and other green fluorescent proteins or dyes having similar characteristics to GFP in combination with a laser beam emitting light of a wavelength of 633 nm in order to excite Cy-5 and other red fluorescent dyes or proteins having similar characteristics to Cy-5. The two laser beams are filtered and directed via the optical setup (comprising at least one dichroic mirror as well as tube and objective lenses of the microscope) of the FCCS device to the microscopic small volume as mentioned above, exciting the corresponding fluorescent molecules in said volume. Preferably, two different species of fluorescent molecules with GFP and Cy-5 being most preferred, are excited via said two laser beams (in case of GFP and Cy-5 with wavelengths of 488 nm and 633 nm, respectively).

Fluorescent molecules emit fluorescence upon excitation and said emitted fluorescence is split again subsequently for detection via the optical setup of the FCCS device being comprised of dichroic mirrors and filters. Finally, said fluorescence is detected in the corresponding detection channels.

Thus, information on the diffusion of single fluorescent molecules of two different types within the microscopic small detection volume is detected. The corresponding correlation curves are preferably averaged from 5 measurements (10 s each) of each sample. For each type of fluorescent molecule, said information of diffusion comprises *inter alia* information on the number of molecules and the speed of the molecules.

Said information is then analyzed by software which is available in combination with FCCS devices. Any appropriate software available may be used in order to determine interaction parameters according to the invention. The person skilled in the art is aware of such analysis software and no further modification to the software may be necessary.

In the following, the main steps of said analysis will be explained in a very simplified view.

The process of diffusion of fluorescent molecules within said microscopic small volume leads to the detection of fluorescent fluctuations over time which may be depicted in a graph as fluorescence fluctuations over time. As mentioned above, said diffusion fluctuations depend *inter alia* on the number of molecules and their speeds.

By a mathematical process called "autocorrelation analysis" it is possible to extract information from the signal fluctuations as mentioned above. In a simplified view, the events detected and expressed in the signal fluctuation-pattern are shifted over time in order to find recurring events and patterns in said signal fluctuation pattern. The result is a so called autocorrelation curve consisting of the derivation of G over time τ [G(τ)] on the y-axis and the autocorrelation time [τ] on the x-axis.

It is possible to deduce several parameters from said autocorrelation analysis: *inter alia* the number of fluorescent molecules in a defined volume and thus the concentration of the fluorescent molecule, the diffusion speed corresponding to the size and the molecular weight as well as the determination of different subpopulation (e.g. small vs. big, slow vs. fast,...). Data on different subpopulations may be of particular relevance if the specificity of the binding between two analytes should be determined. If a compound displays a high affinity to a protein (corresponding to an "on-target"-effect) but also binds to a large number of further proteins as determined by the presence of many different subpopulations (corresponding to an "off-target"-effect), said compound may not be an ideal candidate for a pharmaceutical application.

When working in a purified two component system wherein one component is fluorescently labelled, it is possible by autocorrelation to determine the fraction of the unbound fluorescently labelled compound and the fraction of fluorescently labelled compound bound to the second component, which is the only further component present in said purified two component system.

However, in case of the present invention, the analysis is done in a huge background of other components due to the nature of the sample in order to work in an *in-vivo-*like environment. Clearly, there will be a huge number of complexes comprising several different molecules (such as proteins or nucleic acids and so on) and the fluorescently labelled component. Said huge number of subpopulations cannot be resolved by autocorrelation. Even if the labelled compound would bind to only two different molecules in said setup with said two different molecules having identical molecular weights, a discrimination between said two different complexes would not be possible by autocorrelation.

In order to overcome said limitation, a second fluorescent label differing from the first label is introduced into the system and the second interaction partner is tagged by said second label. In the following, reference is, however, made to two different fluorescent molecules only.

First of all, at least two different and distinguishable (i.e. with respect to their excitation and emission spectra) fluorescent molecules should be used together in a sample. Thus, if the interaction parameters of e.g. two analytes are determined wherein each analyte comprises a fluorescent tag, said two fluorescent tags preferably show very little or more preferably no fluorescence resonance energy transfer at all. Preferred combinations of fluorescent molecules and/or dyes and/or tags will be mentioned below when discussing preferred embodiments of the invention.

By autocorrelation analysis for each of the fluorescent molecules, it is possible to determine several parameters as set out above, *inter alia* the individual concentrations of both of said molecules in the detection volume.

The corresponding two autocorrelation graphs G(τ) over (τ) may then be compared in the so called "cross correlation analysis", and, it is then possible to *inter alia* deduce identical events and patterns in both of said graphs. Said results, however, represents the information for complexes comprised of said two fluorescent molecules (and optionally further molecules). Thus, by the cross correlation analysis, it is possible to determine *inter alia* the concentration of complexes of the two fluorescent molecules. In addition, it is possible to quantitatively determine the "on-target" fraction (i.e. a complex of the two labelled analytes) as well as the "off-target" fraction (i.e. other complexes). This result represents important information on the specificity of the binding.

Thus, the single concentrations of both molecules as well as the concentration of complexed molecules are known via the two correlation analyses. With the definitions above for Kd = [P] [C] / [PC] and [P], [C] and [PC] known, the Kd-value can be determined.

In order to rely on several measurements and to do the analysis within the linear range of the reaction (wherein neither of the analytes is limiting), one of the fluorescently labelled interaction partners may be titrated vs. a constant concentration of the second fluorescently labelled interaction partner. About at least 50, 40, 30, 20, 15, 12, 11, 10, 9, 8, 7, 6 or 5 dilution steps may be used for said titration wherein the steps are preferably prepared by pipetting from one well to another to limit pipetting errors.

Thus, in case the Kd-value should be determined for the interaction between e.g. a protein fused to GFP and a compound labelled with Cy-5, 12 samples (each one comprising 20 µl except the first comprising 40 µl) taken from the lysate comprising said GFP-fusion protein may be pipetted into 12 wells of a 384 well plate. Subsequently, about 0.2 µl of the Cy-5 labelled compound may be pipetted to the first well, followed by the transfer of 20 µl from said well to next and so on. For each well, the FCCS analysis comprising auto- and cross correlation may then be performed and the Kd-value may be determined within the linear range of the 12 results gained. Such an analysis may be done in a short time comprising between about 3 and 30 minutes with 20 minutes being preferred.

However, it is also possible to determine the kinetic parameters of an interaction between two fluorescent molecules and differently fluorescently-labelled interaction partners, respectively.

This represents a major advantage over other systems, as the knowledge of the kinetics of a binding reaction may lead to the identification of very tight binding partners, which, however, have a slow kinetic of binding.

This will be exemplified in the following: An inhibitor may display a high binding affinity towards an enzyme resulting in the inhibition of said enzyme. However, the kinetic of the reaction may be very slow (thus, the inhibitor may be referred to as "slow binder"), e.g. 1 h till saturation of the enzyme. In a standard enzymatic test in order to screen for inhibitors, the incubation time may, however, be only 10 minutes in a purified system. In this case, the inhibitor may not be selected for further studies as it displays only weak inhibitory activity. However, *in vivo*, the exact same inhibitor may be a strong inhibitory compound due to other cellular factors present but with a slower binding kinetic. Often, so called "backpocket binders" are slow binders displaying a high efficacy due to their long retention time.

In order to determine the kinetic parameters of an interaction, the exact same setup as described above may be used. However, the sample comprising both fluorescent molecules or fluorescently-labelled compounds, respectively, may be subjected to measurements at different time points of the incubation, e.g. every 2 minutes over a total period of 5 hours. Thus, a kinetic of said reaction is recorded. Time points may also be chosen at every about 1, 3, 4, 5, 10 or 20 minutes of the reaction over a range of about 1, 2, 3, 4, 6, 7, 8, 9 or 10 hours. Depending on the interaction analyzed, it may even be recorded for a longer or for a shorter period.

In case the Kobs-value should be determined for the interaction between e.g. a protein fused to GFP and a small molecule compound, not being a protein, a peptide or a nucleic acid, labelled with Cy-5, 4 samples (each one comprising 30 µl) taken from the lysate comprising said GFP-fusion protein may be pipetted into wells of a 384 well plate. 10 µl of Cy5-labelled small molecule compound, not being a protein, a peptide or a nucleic acid, may then be added to each well at four different concentrations and each data point may be averaged from 4 individual measurements over 5 seconds. The data points may be recorded over a period of 5 hours every two minutes.

With the concentration for the complexes known for several time points by cross correlation analysis as well as with the kobs-values determined by following the complex reaction over time by cross correlation analysis, it is possible to determine the kinetic parameters kon and koff with kobs = kon x [PS] + koff as outlined above.

With Kd = koff / kon, and koff and kon known, it is again possible to determine the Kd-value, this time via the kinetics of the reaction. This alternative way of determining the Kd-value of course introduces a major advantage into the analysis as performed, as one value is determined by two different approaches.

Before describing the other steps carried out for the method of the present invention, the determination of interaction parameters for a competitive agent, being a small molecular compound, will be explained shortly in the following.

For determining the binding affinity of a competitive compound towards a target, said competitive compound does not need to be labelled. Before the analysis, however, the cross correlation parameters of two fluorescently labelled interaction partners need to be determined, exactly as set out above. To said existing interaction, a competitive compound is added over a concentration range. This may be done e.g. by adding the competitor in 12 different concentrations to the already established interaction between a target and a small molecular compound via serial dilution to the wells. Then, the cross correlation parameters of the two fluorescently labelled interaction partners are determined again. Such an analysis may be done in a short time comprising between about 10 and 60 minutes with 45 minutes being preferred.

The amplitude of cross correlation will now be influenced by the competitor in a concentration dependent manner, and said relationship may be expressed as the amplitude of the cross correlation on the y-axis vs. the concentration of the competitor on the x-axis. From said graph, one can determine the concentration of the inhibitor, at which the amplitude corresponds to the IC50-value (0.5 x the initial height of the amplitude).

Finally, as outlined above, with the Cheng-Prusoff equation Ki = IC50 / (1 + [L] / Kd), the Ki-value of the competitor may be determined. Said Ki-value corresponds to the Kd of the interaction between the competitor and the target. By this easy and fast way of determining Ki-values, a large group of competitors may be screened.

As above, said Ki-value may also be determined via the kinetic of the competitive reaction, namely by the kon and koff values of the competitive agent and the target, a protein of pharmaceutical interest.

For determining the kon-value of the competitor, the cross correlation parameters of the two fluorescently labelled interaction partners need to be established first and, after addition of the competitor, the kinetic of the reaction is recorded. To the established interaction, a dilution series of the unlabelled competitor (e.g. 4 different concentrations) is added and cross correlation parameters are determined at several time points (e.g. every 3 minutes over a time period of 4 hours). The cross correlation amplitude will be influenced by the competitor over time and may be depicted in a graph of said amplitude on the y-axis vs. time on the x-axis. By fitting said graph according to y = y_{∞} + y₀ x exp^{(-kobs)xt}, kobs may be determined and depicted in a graph on the y-axis with the concentration of the inhibitor on the x-axis. After applying linear regression , the kon- and Koff-values for the competitor can be determined with kobs = kon [K] + koff.

However, for accuracy reasons it is preferred to determine the koff value separately. To this aim, the unlabelled competitor needs to be preincubated with the labelled target in order to establish a complex. To said existing complex, the labelled small molecular compound (which is already known to interact with the target) is added and competing for binding with the competitor. Again, said reaction is followed over time by corresponding determination of cross correlation parameters. In order to determine the koff value, the amplitude of the cross correlation analysis may be depicted on the y-axis vs. time on the x-axis. By fitting said graph according to y = (y_{∞} - y₀) x (1 - exp^{(-kobs)xt}) + y₀, kobs may be directly determined.

Finally, with Ki = koff / kon, the Ki-value of the competitor may be determined. Again, this alternative way of determining the Ki-value of course introduces a major advantage since a single value may be determined by two different approaches.

It needs to be understood that all volumes of samples used for the measurements mentioned above can be adjusted according to the purpose of the measurements and the samples provided, i.e. the lysate and the labelled analytes. The skilled person knows how to adjust concentrations, dilution steps and the like. However, the volume of a sample used according to the present invention does not fall below a minimum volume of 5 µl.

According to another step of the method of the present invention, cells cultured outside the human or animal body are lysed in order to obtain a sample.

In principle, cells of any origin, which are routinely used in laboratories may be used for the present invention. Thus, cells from archeae, bacteriae such *E. coli,* yeasts such as *S*. *cerevisiae* or mammalian cells may be used with mammalian cells being preferred. Said mammalian cells can be selected from the group of mammalian cells comprising HEK 293, HEK 293 T, HeLa and HUVEC cells. Preferably, HEK 293 cells are used.

It should be noted that different cell types (e.g. of different human tissues or of different differentiation types within a tissue) can be used for the present invention with the consequence that interaction parameters of the at least two analytes can be determined in specific environments. This introduces a main advantage to the system since it is possible to e.g. determine interaction parameters for a small molecular compound and a target in a lysate of a specific cell type (such as human skin cells or human kidney cells or the like) where the compound may eventually be applied.

With respect to the analysis of interactions in different environments it should be mentioned that the present invention allows for said analysis in different backgrounds. Thus, an interaction between a labelled small molecular compound and a labelled protein in a lysate of human cells (wherein the cells were transfected with a construct coding for said protein) may be bridged by certain factors present in said lysate, e.g. by other human proteins. Alternatively, the interaction may have been found using pull down strategies with lysates of human cells wherein said bridging factors are, however, also present. In order to exclude said bridging factors, the human protein of interest may be expressed in a different species, such as *E. coli,* by transforming *E*. *coli* cells accordingly, followed by an analysis according to the present invention in the *E.coli* lysate. Since the human bridging factors are not present in *E.coli,* the result can provide additional information on said interaction (e.g. show that the small molecular compound and the protein are not interacting in said different background). Such an analysis may also be done in *S.cerevisiae* or any other organism which may be transfected with constructs coding for human proteins.

The cells may be cultured according to standard procedures known to the skilled person. Mammalian cells may be cultured according to standard cell culture conditions comprising appropriate medium with FCS and standard incubation conditions (such as e.g. about 37°C and about 5% CO₂).

The lysate of the cells prepared as outlined below comprises at least one of said analytes before another one is added. However, after preparing the lysate, the first analyte may also be added (thus, all of the at least two analytes are added in this embodiment) in order to determine interaction parameters in an *in vivo* like environment. Such a sample may be regarded as reconstituted cellular system.

However, it may be preferred to have at least one analyte already present in the cells prior to the lysis by expression of the analyte (a protein) in the cells. For this purpose, the cells may be transformed or transfected with a DNA-construct encoding said protein and incubated for an appropriate time in order to allow for expression of said protein. Any vector known to the skilled person may be used together with any appropriate transformation or transfection technique.

Said protein is a fusion protein comprised of target protein and a fluorescent tag. Said tag is an autofluorescent protein.

If the analyte is expressed in the cells, it is preferred to use standard transfection methods such as calcium-phosphate-precipitation or carrier-based techniques such as lipofectamin, effectene or the like for transfection. However, one may also use electroporation techniques or the like. Any protocol known to the skilled person may be used for this purpose.

The expression of the DNA-sequence encoding the labelled analyte, e.g. a GFP-fusion protein, may either be transient or stable.

If transient expression is chosen, cells are cultured for additional about 6 hours to about 48 hours after transfection according to standard conditions to allow for expression of the said DNA-sequence.

If stable expression is chosen, a vector comprising the DNA-sequence coding for the labelled analyse, a marker for selection (such as a Neomycin-resistance gene or the like) is selected for transfection. A variety of such vectors is known to the skilled person and any appropriate vector may be used. Following transfection, cells are selected (in the above example by adding Neomycin) a few days after transfection for another about 3 to about 5 days to select for stable integration.

In both cases, the vector used may comprise an inducible promoter (e.g. inducible by addition of tetracycline or inducible by the absence of tetracycline [Tet on/ Tet off system]). The expression can thus be induced, e.g. by adding tetracycline. An inducible expression system may be preferred for to the present invention since it allows for controlling the amount of labelled analyte. As said amount should reflect the *in vivo* expression level of the analyte, the analyte should not necessarily be overexpressed.

Before lysis of the cell, an aliquot of the cells expressing the labelled analyte may be assayed for analyte-expression, e.g. by Western blot. In said Western blot, a fluorescent protein-part of the fusion protein may preferably be detected since antibodies are available against this part and the fluorescent protein it is usually not expressed in cells. Any other method may be used as well; this quality control step represents a routine step for the skilled person.

According to the present invention, the cells mentioned above are lysed in order to provide a lysate as sample. All steps are preferably carried out at 4 °C with corresponding buffers preferably also at 4°C. The lysis protocol may in a preferred embodiment comprise the following four main steps: addition of hypotonic buffer, breaking open of cells using mechanical force, restoration of physiological salt conditions and ultracentrifugation.

Before adding the hypotonic buffer, the cells are collected and washed by standard methods comprising detaching of cells by adding e.g. PBS and/or using trypsin/ EDTA and one or optionally several wash-steps using e.g. PBS and centrifugation steps to pellet washed cells.

The hypotonic buffer added may be a regular Tris/HCl-buffer at an appropriate concentration such as 2, 3, 4, 5, 6, 7, 8, 9, 10, 11, 12, 13 , 14, 15, 16, 17, 18, 19, 20 mM with 10 mM being preferred at a physiological pH. Low salt such as 0,5 mM MgCl₂ may be present in addition to low concentrations of EDTA (such as about 5 mM) and protease as well as phosphatase inhibitors. However, said buffer does not comprise physiological NaCl concentrations or the like and, therefore, is hypotonic compared to the salt concentrations within the cells. This will result in swelling of the cells along with the lysis of some of the cells. However, swelling of the cells will be the main effect. For this purpose, cells may be incubated for several minutes on ice with 5 minutes being preferred. Swelling of the cells may be controlled by microscopy. It is preferred that the hypotonic buffer does not comprise a detergent. However, if a detergent is comprised in the hypotonic buffer, its concentration is below its CMC as set out below.

In order to break the swollen cells open, mechanical force, preferably shear force is used. A glass douncer with a tight fitting pestle (to create shear force) may be used; thus, the cell suspension comprising the swollen cells may be transferred to the douncer and said suspension may be homogenized by about 20 strokes of the pestle. However, any other technique employing mechanical force resulting in lysed cells may be used as well.

Buffer to restore physiological salt conditions may then be added; this step assures that the FCCS measurements are taken under *in vivo* like conditions. Physiological salt concentrations may be essential for *inter alia* the correct folding of certain proteins, which may be targets. Tris/HCl buffer as outlined above may be added comprising, however, NaCl and the like at a concentration appropriate to restore physiological salt concentrations in the final sample.

Finally, the lysate may be spun in an ultracentrifuge, preferably at 150 000 x g for 2 hours at 4°C in order to get rid of cell debris. However, a regular centrifugation at lower speed may also be used. The resulting supernatant represents the sample, to which glycerol and TritonX-100 to a final concentration of 5% and 0.1 mM, respectively, may be added.

In certain embodiments of the invention, the protocol may comprise two main steps only, namely breaking open of cells by methods such as using mechanical force and/or sonification followed by ultracentrifugation wherein buffer comprising physiological salt concentration is used throughout the protocol.

In general, other lysis methods comprising the use of detergents may be used as well as long as the concentration of any detergent is below its critical micellular concentration (CMC). In one embodiment, a detergent below its CMC without the use of mechanical force may be applied. Detergents which may be used according to the present invention are listed below with their corresponding CMCs. The term "critical micellular concentration" defines the concentration of a detergent above which micelles are spontaneously formed. Preferred detergents are selected from the group of detergents comprising (wherein the CMCs are given in mM at 25°C) Triton X-100 (with a CMC of about 0,3 mM), Tween-20 (with a CMC of about 0,08 mM), Nonidet-P40 [also referred to as NP-40] (with a CMC of about 0,25 mM) and CHAPS (with a CMC of about 4,5 mM, wherein the CMC of CHAPS seems to be dependent on the salt concentration: the CMC in the absence of any salt is about 6,4 mM, whereas the CMC is about 4,1 mM in the presence of 1,5 M NaCl).

According to the present invention, at least two fluorescent labelled analytes (claims 1-3) and one competitive agent (claims 2 and 3) need to be provided. As mentioned above, one of said fluorescent labelled analytes is a fusion protein comprised of the target protein and an autofluorescent protein. A second fluorescent labelled analyte is a small molecule compound. In the following, labelling of said second analyte will be described.

The second fluorescent label may preferably be a fluorescent dye selected from the group of fluorescent dyes comprising Cy3, Cy5, texas-Red, Alexa-dyes and the like, with Cy5 being preferred. Any commercially available Cy5 dye may be used, e.g. Cy5 provided by GE Healthcare Amersham. Said commercially available dyes are already prepared for coupling reactions and, thus, the second analyte, a small molecule compound, may only be added to the ready-to-use mix according to the manufacturer's protocol. Said coupling reaction may be performed overnight under conditions wherein the reaction is protected from light in order to minimize side reactions induced by light. Such coupling reactions are routine work for the person skilled in the art and may for example proceed via the formation of succimidylesters.

After coupling, said label is covalently linked to the analyte. In preferred embodiments of the invention, said fluorescent labelled analyte is then purified via HPLC in order to get rid of analyte and label, which have not reacted. Any HPLC method known to the skilled person may be used. However, it is preferred to do the HPLC purification on a reversed phase matrix using a MeCN gradient (e.g. from about 30%-40%, 35%-45%, 40%-50%, 45%-55% or 50%-60%). Furthermore, it is preferred to follow the corresponding peaks via two detection channels, the first being a UV-channel and the second being a channel suitable for detection of the maximum absorption wavelength of the fluorescent label. Thus, in the UV-channel, one may observe three different peaks at retention times corresponding to free analyte, free fluorescent dye as well as to covalently linked analyte-dye. In the channel of the absorption maximum of the fluorescent dye, one may observe two peaks only corresponding to free fluorescent label and fluorescent label-analyte. However, the retention times of the two peaks will correspond to the retention times observed in the UV-channel for the free fluorescent label and the complex, respectively. Thus, one may definitely identify the peak of the analyte covalently linked to the fluorescent label and collect the corresponding fractions.

When collecting fractions of the complex peak, one may rely on very little material only and concentrate on material eluting in the middle of said complex peak. Thus, it can be assured that only highly purified complex of the analyte and the fluorescent label is collected and used for further studies.

In order to have a second validation of the identity of the complex consisting of the analyte and the fluorescent dye, one may do a mass-spec analysis of said eluted material. With knowledge of the molecular weights of both the analyte and the fluorescent label, one may easily determine by mass-spec whether said reaction is complete and whether said complex corresponds to the expected complex. For this purpose, any mass-spec device may be used with any protocol known to the person skilled in the art. However, any other method suitable for quality control analysis may also be used.

Prior to any analysis, one may dry the fraction via a vacuum concentrator and via lyophilisation overnight. Following said steps, one may then dissolve the dried complex of in appropriate solvent, e.g. DMSO or 50% MECN-dH₂O. Preferably, complex used in FCCS studies is dissolved in DMSO whereas a further aliquot of the complex may be dissolved in 50% MECN-dH₂O for MS-analysis.

At the end of the protocol as described above, one may have a fluorescent dye labelled analyte in an appropriate solvent such as DMSO at hand, typically in a volume of 40 µl. With an estimated molecular weight of 1 kDa for a typical small molecule compound and a concentration of said analyte of 5 mM in the final sample, the amount of labelled analyte is by far enough for the FCCS measurements wherein 1 µg of labelled analyte can be used for 100,000 single measurements. Thus, the amount of the fluorescent labelled analyte is by far enough for performing e.g. high performance screens. Correspondingly, the amount of fluorescent dye as well as analyte used in the coupling reaction may be kept low in order to save material and costs.

The above procedure has been described with respect to an analyte being a small molecule compound labelled with a fluorescent dye such as Cy5. However, said analyte may be tagged with any other fluorescent label as outlined above.

Of course, one may also use coupling reactions *in vivo* as known to the skilled person. Coupled analyte may then be purified from a lysate of the cells used for expression and added in the purified form the lysate.

In summary, it is thus possible to determine interaction parameters of at least two analytes in an *in vivo* like setup, wherein said two analytes may be selected, within the scope of the claims, from a broad variety of molecules, optionally with at least one further competitive agent. Therefore, the methods of the present invention display a broad application range.

In the following, examples of the methods of the present invention are outlined. However, said examples should not be construed as limiting the scope of the present invention, which is defined by the appended claims.

### Examples

### Example 1: Labelling of small molecular compounds

### Cy5 labelling of PD-173956

To label a 10 mM DMSO solution of PD-173956 with Cy5, a 50 µl aliquot was transferred to a tube comprising approximately 250 nmol of monofunctional Cy5 (GE Healthcare Amersham Cy5 Mono reactive Dye Pack). 0.8µl of DIEA (Di Iso Propyl Ethylamin) were added and compound and dye were allowed to react over night at room temperature on a head over tail (HOT) incubator. The reaction vial was covered with aluminum foil to protect the dye from exposure to light during reaction.

Typically, probes carrying a linker with a terminal reactive aminogroup (such as PD-173956) were linked to functionalized Cy5 monofunctional dye (GE Healthcare Amersham, UK) via its reactive NHS ester group.

The purification of the reaction product was typically carried out on a HPLC using a reverse phase column.

PD-173956-Cy5 was purified on a reverse phase HPLC, using a MeCN-dH₂O-TFA (0.1 %) gradient of 45 %-55 % MeCN. The purified product fractions were predried on a vacuum concentrator and lyophilized over night. Parts of the dried Cy5-labelled compound were dissolved both in DMSO (for determination of the concentration and FCCS measurements) and in 50% MeCN-dH₂O (to analyze the purity by mass spectroscopy).

### Cy5 labelling of BAY 43-9006

To label a 10 mM DMSO solution of BAY 43-9006 with Cy5, a 50 µl aliquot was transferred to a tube comprising approximately 250 nmol of monofunctional Cy5 (GE Healthcare Amersham). 0.8 µl of DIEA were added and compound and dye were allowed to react over night at room temperature on a HOT incubator. The reaction vial was covered with Aluminum foil to protect the dye from light during reaction.

BAY 43-9006-Cy5 was purified on a reverse phase HPLC, using a MeCN-H₂O-TFA (0.1 %) gradient of 40 %-50 % MeCN. The purified fractions were predried on a vacuum concentrator and lyophilized over night. Parts of the dried Cy5-labelled compound were dissolved as well in DMSO for determination of the concentration and FCCS measurements as in 50% MeCN-H₂O to analyze the purity by mass spectroscopy.

### Example 2: Labelling of target proteins

### Cloning of bRAF as N-terminal GFP fusion in pGTOc-attR

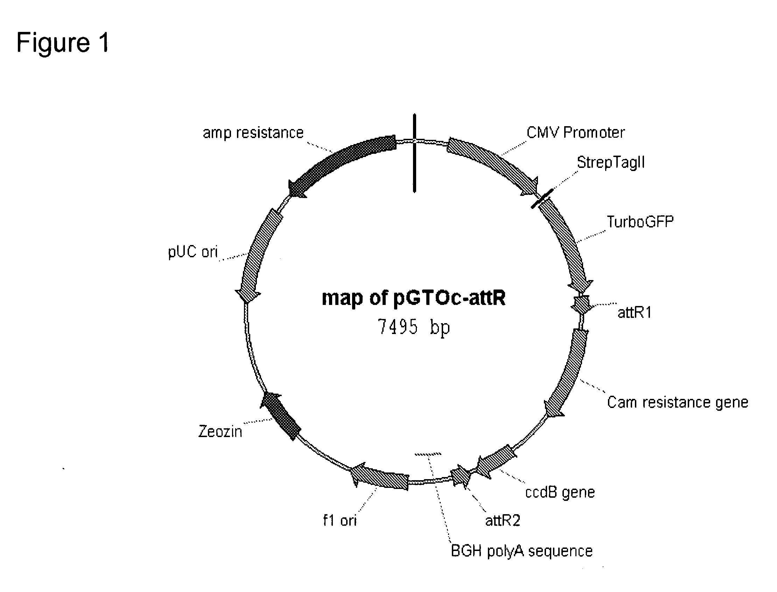

An appropriate vector called pGTOc-attR encoding Turbo-GFP (Evrogen) as N-terminal fusion was designed and constructed by standard methods. The plasmid map of pGTOc-attR is depicted in Figure 1 and the sequence of pGTOc-attR corresponds to SEQ ID No. 1. The pGTOc-attR vector is based on pcDNA4/TO (Invitrogen) and carries TurboGFP (Evrogen), a Strep-TagII (IBA) and a Gateway shuttling cassette (Invitrogen). pGTOc-attR is a 7495 bp vector that upon insertion of the gene of interest via Gateway cloning expresses the gene of interest as N-terminal GFP fusion gene under control of a hybrid CMV/TetO₂ promoter with the following main features: CMV promoter; CMV Forward priming site; Tetracycline operator sequences; StrepTagII; TurboGFP gene; attR1; Cam resistance gene; ccdB gene; attR2; BGH Reverse priming site; BGH polyA signal; F1 origin; SV40 early promoter and origin; EM-7 promoter; Zeocin resistance; SV40 early polyA signal; pMB1 origin; *bla* promoter; ampicillin (*bla*) resistance gene.

The coding sequence of the catalytic domain of bRAF corresponding to amino acids 433 to 767 (see SEQ ID No. 2 for nucleotide sequence) flanked by attB-sites (in vector pACT2-HA-att; see Gateway^{™} manual) was shuttled into pGTOc-attR via Gateway^{™} cloning according to the Gateway^{™} manual.

The following fragments of the plasmid sequence of pGTOc-bRAF show the vector assembly for the shuttled catalytic domain of bRAF:
SEQ ID No. 3: Normal font : TurboGFP coding sequence
   Underlined: attB1 site
   *Italics: linker sequence*
   **Bold**: bRAF coding sequence starting with nucleotides for aa 433
SEQ ID No. 4: **Bold**: bRAF coding sequence ending with nucleotides for aa 767
   *Italics: Stop codon and linker sequence*
   Underlined: attB2 site
   Normal font : adjacent vector sequence

After controlling the shuttling step by standard methods comprising sequencing, it was confirmed that the coding sequence of the catalytic domain of bRAF was present in pGTOc-attR 3' to and in frame with the coding sequence of TurboGFP resulting in pGTOc-bRAF.

Plasmid-DNA of pGTOc-bRAF was prepared in µg-amounts by conventional techniques (amplification in *E. coli* followed by plasmid preparation using the Qiagen DNA-Maxi Preparation kit).

### Transfection of_{.}pGTOc-bRAF into T-REx^{™}-HEK 293 cells (Invitrogen)

T-REx™-293 cells from Invitrogen, Germany, were used for expression of the fusion protein. The T-Rex-system allows for an induction of gene expression via tetracycline; based on the TET-ON System, expression in T-REx™-293 cells can be induced by the addition of tetracycline. T-REx™-293 cells are stably expressing pcDNA6/TR and are routinely grown under blasticidin (Invitrogen, Germany) selection in DMEM (Invitrogen, Germany) plus 10% fetal calf serum (PALL Life Sciences Biopharmaceutical, UK), 2 mM Na-pyruvat (Invitrogen, Germany) and 2 mM glutamine (Invitrogen, Germany).

24 hours prior to transfection, cells were seeded in at least two 10 cm cell culture dishes at 2x10⁶ viable cells per dish. Cells were maintained at 37°C and 5% CO₂ humidified air until they were semi confluent. The cells were then transfected with 2 µg of plasmid DNA per dish using Effectene (Qiagen, Germany). Transfection was done as described by the manufacturer's guidelines.

To induce expression of GFP-bRAF, cells were exposed to 0.2 µg/ml tetracycline 38 hours after transfection. 6 hours after induction, expression of GFP-bRAF was monitored prior to harvesting and lysing the cells. Expression of GFP-bRAF was monitored by fluorescence microscopy and the intracellular distribution of GFP-fusions, cell viability and percentage of expressing cells was documented.

### Preparation of cell lysates

All steps were carried out on ice and with solutions at 4°C. The adherent transfected cells were rinsed once with D-PBS (w/o Ca and Mg; Invitrogen, Germany) before adding 5 ml of D-PBS per 10 cm cell culture dish. Under such treatment cells were detached from the surface and could be harvested by pipetting after 10 min. Cells were centrifuged gently at ~2000 x g (5 min, 4°C) and the resulting pellet was resuspended in 10 ml D-PBS. After centrifuging another time at -2000 x g (5 min, 4°C) cells were resuspended in cold Dounce-buffer (1.125 ml per cell pellet of each 10 cm cell culture dish) containing
- 10 mM Tris/HCl, pH 7.6,
- 0.5 mM MgCl₂,
- 5 mM EDTA,
- Complete protease inhibitor cocktail (Roche Applied Science, Germany)
- phosphatase Inhibitor cocktail 2 (Sigma-Aldrich, Missouri, USA).

Within 5 min on ice, cells underwent hypotonic swelling. After swelling was confirmed by microscopy, the cell suspension was transferred into a glass douncer with a tight-fitting pestle and homogenized with 20 strokes.

To restore physiological salt concentration 0.375 ml tonicity buffer consisting of
- 10 mM Tris/HCl, pH 7.6,
- 0.5 mM MgCl₂,
- 5 mM EDTA,
- 0.6 M NaCl,
- Complete protease inhibitor cocktail (Roche Applied Science, Germany)
- phosphatase Inhibitor cocktail 2 (Sigma-Aldrich, Missouri, USA)
was added and the suspension was mixed gently. The lysate was transferred into an ultracentrifuge tube and centrifuged at 150000 x g for 2 hours at 4°C. The resulting supernatant was supplemented with 5% glycerol (final concentration) and 0.1 mM TritonX-100 (final concentration) and either used directly or frozen at -70°C. Lysing cells of two 10 cm cell culture dishes according to the above protocol resulted in a ready to use cellular lysate of a total volume of 3 ml.

An aliquot of the lysate was the loaded on an SDS gel and analyzed for presence, size and integrity of the GFP-fusion protein by western blotting using an anti-GFP antibody directed against Turbo-GFP. SDS-PAGE and western blotting were performed using standard procedures.

### Example 3: FCCS measurements

FCCS measurements were performed on a ConfoCon®2 system (Carl Zeiss, Germany). For excitation of the GFP and Cy5 fluorophores, the 488 nm laser line of the argonion laser and the He-Ne 633 nm laser line, respectively, were used. The two pinholes and the cross-correlated volume element were adjusted by calibration measurements according to the manufacturer's protocol. In short, said adjustment is based on focusing on a pinhole in a first sample comprising fluorescent dye excited by the blue laser and determining the emission maximum followed by the determination of the emission maximum of a second sample comprising fluorescent dye excited by the red laser in the same pinhole. All solutions were prepared in Dulbecco's Phosphate-Buffered Saline (D-PBS) (Invitrogen, Germany) or Hanks Buffered Saline (HBS) (Invitrogen, Germany) plus 5% glycerol. Samples of 20 µl to 50 µl were pipetted in wells of 384-well microtiter plates (MMI GmbH, Germany) and kept on ice for at least 30 min. The microtiter plate was then fixed onto the microscopic stand which was set on an objective lens (C-Apochromat 40× 1.2W; Carl Zeiss) and allowed to adapt to room temperature. All measurements were performed at room temperature (23°C). Correlation curves were averaged from 5 measurements of 10 s each. The autocorrelation curves and the cross-correlation curves of FCCS data were analyzed by using the fitting algorithms of the software package for ConfoCor®2 (Carl Zeiss).

### Example 4: Determination of interaction parameters of two analytes using FCCS

### Determination of linear range

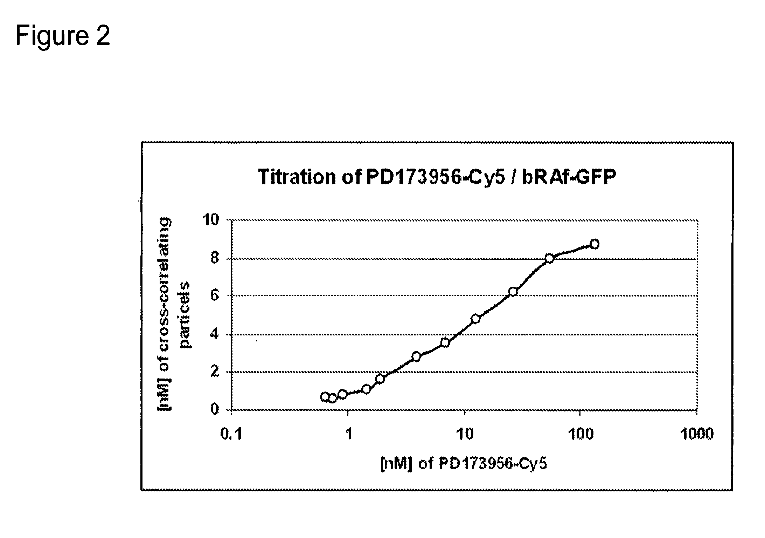

To determine interaction parameters of two analytes, labelled compound was added to the lysate comprising the GFP-fusion of the target (shown for PD-173956-Cy5 and GFP-bRAF in Figure 2). Depending *inter alia* on "off target" effects, only a fraction of added labelled compound is, however, available for binding. Thus, the concentration of a binding partner may be limiting, even though added at equimolar concentrations. To address this, labelled compound was titrated over a concentration range of between 100 nM and 1 nM to aliquots of the lysate. Measurements were carried out and resulting cross correlating particle numbers were plotted against the concentration of labelled compound in logarithmic scale (see Figure 2). In general, data are valid only within the linear range where neither of the binding partners is limiting.

### Determination of affinity parameters

The lysate comprising GFP-bRAF was thawed on ice and diluted in HBS (Hanks buffered saline) + 5% glycerol to give appropriate concentration of GFP-bRAF; particle numbers between 0.5 and 2, which corresponds to concentrations of between 5 nM and 20 nM, gave satisfying signals. About 400 µl lysate dilution was prepared and an aliquot of 40 µl followed by aliquots of 20 µl were distributed into the wells of the 384-well microtiter plate. About 0.2 µl PD-173956-Cy5 was then added to the first well and mixed thoroughly. To gain serial dilutions, repeated cycles of transferring 20 µl from the first well to the next well were performed. After 10 min incubation on ice, the microtiter plate was fixed on the microscopic stand and FCCS measurements were performed.

Correlation curves were averaged from 5 measurements of 10 s each. The autocorrelation curves and the cross-correlation curve of FCCS data were analyzed by using the fitting algorithms. The functions were fitted to one- or two-component models with diffusion times corresponding to those of the fluorescent derivatives. Successful fitting resulted in particle numbers, which can be translated in concentration values for free and bound fractions of the labelled compound, the concentration of available target fusion protein and the concentration of compound-target-complexes. As outlined in the specification, said values are sufficient to deduce the K_{D} by application of the law of mass action.

For the interaction of Cy5-labelled PD-173956 to GFP-bRAF a K_{D}-value of 15 nM to 20 nM was determined. For the interaction of Cy5-labelled Bay43-9006 to bRAF-GFP a K_{D}-value of 20 nM to 30 nM was determined.

### Determination of kinetic parameters

It was observed that Cy5-labelled Bay43-9006 takes longer to bind GFP-bRAF compared to PD-173956-Cy5. Only after incubation of up to 5 hours a constant affinity was measured, indicative for a slower binding kinetic.

To determine binding kinetics in detail, time resolved measurements were carried out. Generally, aliquots of the lysate comprising GFP-target protein were incubated with Cy5-labelled compound at different concentrations and measured in intervals over a period of 1-12 hours for this purpose. Kinetics were observed sufficiently long to deduce binding curves.

As binding of the labelled compound is concentration dependent, the resulting curves exhibit different slopes and thus do not represent the kₒₙ ond k_{off} values but so called k_{obs} -values, with k_{obs} = kₒₙ *c + k_{off}. The resulting curves can be fitted to a function S = B + A c^{(*k*obs)t}.

Plotting the resulting k_{obs} values against the concentration of the labelled compound results in a straight line (an operation called linear regression), the gradient/slope of which indicates the kₒₙ value and the section of the y-axis the k_{off} value, respectively.

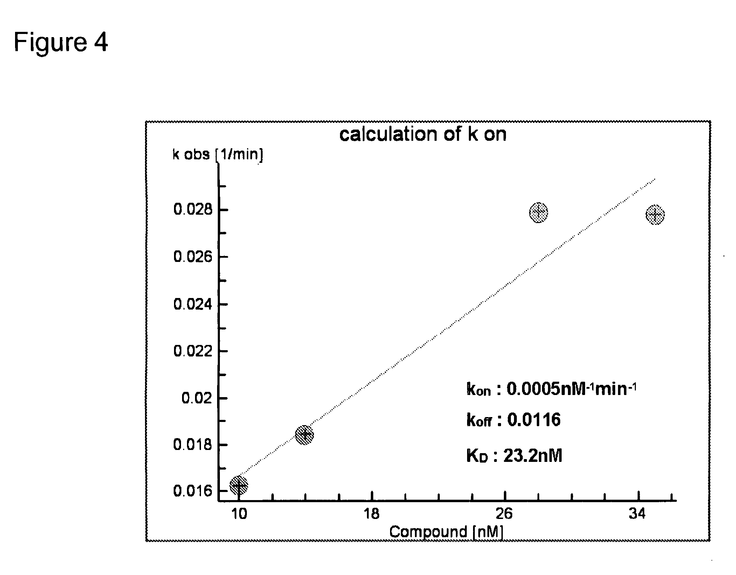

For the kinetic analysis of Cy5-labelled Bay43-9006 binding to GFP-bRAF, Bay43-9006-Cy5 was added at concentrations of 35 nM, 28 nM, 14 nM and 10 nM, respectively, to aliquots of the lysate comprising GFP-bRAF (see Figure 3). Data points were recorded for each concentration at an interval of 2 minutes over a total time period of 320 minutes. Each data point was averaged from 4 individual measurements over 5 seconds. Binding curves representing k_{obs} - values were analyzed as described above. A kₒₙ rate of 0.0005 nM⁻¹min⁻¹ and a k_{off} rate of 0.0116 min⁻¹ was calculated, which translates into a binding constant of 23 nM (see Figure 4).

### Example 5: Determination of interaction parameters for one competitive agent influencing the interaction of two analytes using FCCS

### Determination of affinity parameters

To measure the affinity of unlabelled agents or compounds, competition experiments were performed in which the labelled analyte is displaced from its target. Thus, an interaction between the target and a labelled compound was established first, resulting in complexes of the two labelled interaction partners. Addition of compounds competing for the binding pocket of the target will displace the labelled compound from the target in a concentration dependent manner. Titrating the competitive compound over a concentration range to the established interaction will result in a gradual decay of the cross correlation amplitude (dependent on the affinity of the competitor for the binding pocket). Plotting the measured amplitudes against the competitor concentration will result in IC₅₀ values that can be translated in Ki-values by application of the Cheng Prusoff equation (Weisshart et al. Current pharmaceutical Biotechnology, 2004, 5, 135-154) The Ki-value is equivalent to the K_{D} -value of the competitor for the target.

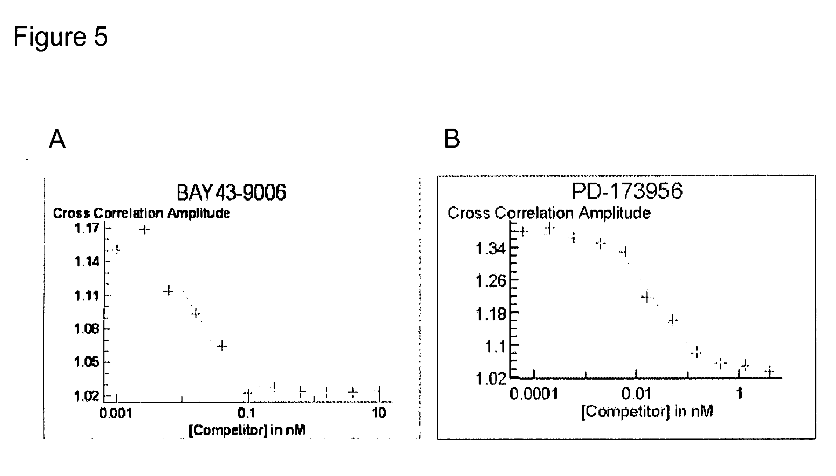

Based on the interaction of Cy5-labelled PD-173956 to GFP-bRAF, competition experiments using either unlabelled PD-173956 or unlabelled BAY43-9006 were carried out (see Figure 5). Labelled Target (GFP-bRAF) and labelled compound (PD-173956-Cy5) were mixed at equimolar concentrations at about 5 nM to 10 nM each and incubated for about 10 to about 30 minutes. Serial dilutions of the unlabelled competitive agents, in both cases ranging from 1 nM to 10 µM were added to the established interaction of the two labelled analytes and subjected to FCCS measurements. Increasing concentrations of both compounds resulted in decreased cross correlation amplitudes. Amplitudes were plotted against the competitor concentration to deduce the IC₅₀ -value for both competitions and fitted as described above (see figure 5).

For the competitive agent Bay43-9006, an IC₅₀ of 114 nM was deduced after 2 hours of incubation. After incubating for 5 hours, an IC₅₀ of 18 nm was observed. For the competitive agent PD-173956, IC₅₀ -values of 20 nM were observed both after incubation for 2 and 5 hours. Thus, the competition reaction of Bay43-9006 took longer to reach steady state level compared to PD-173956. This result indicates that the equilibrium for the interaction of PD-173956 to bRAF has already been established after 2 hours. Using the Cheng-Prusoff Equation, Ki-values can be deteremined. The Ki-values for both competitive agents were between 7 nM and 15 nM, indicative of similar affinities to the target, albeit with different kinetics.

### Determination of kinetic parameters

Based on the observation that Bay43-9006 binds to bRAF with slower kinetics compared to PD-173956, time constants for the kinetics of the slow binding reaction were determined. For this purpose, competition experiments were set up as described above and the kinetics of the competing reactions were measured in intervals of several minutes.

It is advisable to compete an interaction with fast kinetics, so that the time constants of this reaction can be neglected. After the competition reactions (wherein the competitive agent is provided at different concentrations) reach steady state, the fitted amplitudes for each competitor concentration are plotted against the incubation time. As binding/competition of the unlabelled competitor compound is concentration dependent, the resulting curves exhibit different slopes and thus do not represent the kₒₙ ond k_{off} values but so called k_{obs} -values, with k_{obs} = kₒₙ *c +k_{off}. The resulting curves can be fitted to a function S = B + A e^{(-*k*obs) t}. Plotting the resulting kobs values (without the negative algebraic sign) against the competitor concentration (linear regression) results in a straight line, the gradient/slope of which indicates the kₒₙ value and the section of the y-axis the k_{off} value. The K_{D} which can be deduced from the law of mass action is also defined as k_{off}/kₒₙ, so that the K_{D} - measurements in steady state should equal the kinetic measurements.

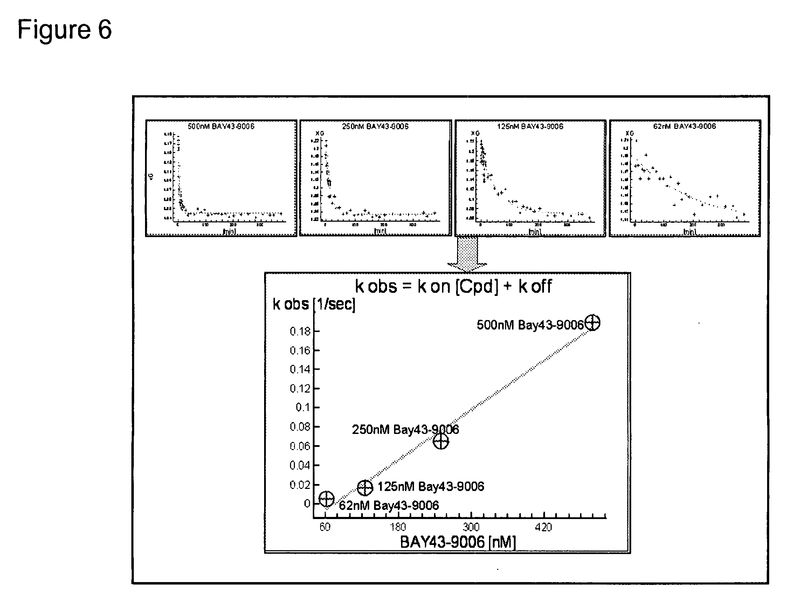

For the interaction of bRAF and Bay43-9006, the competition of the interaction of PD-173956-Cy5 and GFP-bRAF with unlabelled BAY43-9006 was monitored for several competitor concentrations (at concentrations of 62, 125, 250 and 500 nM) over a time period of more than 5 hours (440 minutes, see figure 6) and the following constants were determined: kₒₙ: 0.0005 nM⁻¹min⁻¹ and k_{off}: 0.005 min⁻¹. This translates into a K_{D} of 10 nM which is in agreement with the steady state measurements.

It is, however, advisable to determine the k_{off} value independently since only a minor shift in the slope (and thus minor impact on the deduced kₒₙ value) will have drastic impact on the k_{off}-value represented by the section of the y-axis.

In order to determine the k_{off} value independently, the lysate is preincubated with competitor to allow binding until steady state is reached. Afterwards, an excess of labelled binding partner, ideally with fast binding properties, is added, so that each accessible binding pocket can be occupied by the labelled binding partner. The interaction of labelled partners will result in a gradual increase of the cross correlation amplitude, which represents the decay of complexes comprising target-GFP molecules and unlabelled competitor. Because dissolution is concentration independent, only one concentration of unlabelled competitor has to be measured, to determine the k_{off} value.

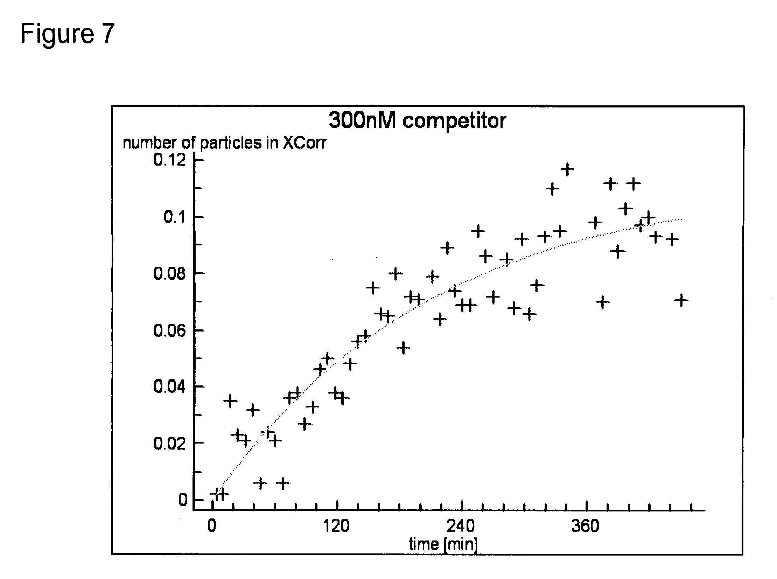

To determine the k_{off} value for unlabelled BAY43-9006 in a complex consisting of GFP-bRAF and BAY43-9006 independently as described above, the two analytes were preincubated overnight (to allow for steady state of the interaction). Afterwards, 300 nM of Cy5-labelled PD-173956 (corresponding to an excess of said compound) was added. Due to the high concentration of PD-173956-Cy5 and its fast binding kinetics, accessible binding pockets of bRAF (from which unlabelled Bay43-9006 has been released) is instantly occupied by PD-173956-Cy5, resulting in a time dependent, concentration independent increase of the cross correlation amplitude (dissociation of compound-target complexes is independent of concentrations, association kinetics of PD-173956-Cy5 can be neglected). The reaction was followed by FCCS over 440 minutes with cross correlating particles being recorded every 3 minutes. By fitting the resulting curve to the function y = (y∞- y₀)*(1-exp^{(-koff)} x)+y₀, the k_{off} value can be calculated (see Figure 7) and corresponded to 0.002 min⁻¹.

## Claims

1. Method of determining interaction parameters of two analytes comprising at least the steps of:
a) providing a fluorescence cross correlation spectroscopy device comprising a loading zone for a sample;
b) providing a lysate of cells cultured outside the human or animal body as sample wherein said lysate comprises the first analyte in the form of a fusion protein comprised of a target protein and an autofluorescent protein;
c) adding the second analyte to the sample of step b);
d) loading the sample comprising said two analytes onto the loading zone of the fluorescence cross correlation spectroscopy device;
e) determining interaction parameters of said two analytes by fluorescence cross correlation spectroscopy;
**characterised in that** the second analyte is a fluorescent dye labelled small molecule compound, not being a protein, a peptide or a nucleic acid.

2. Method of determining interaction parameters for one competitive agent influencing the interaction of two analytes comprising at least the steps a) to e) of claim 1 and further comprises the steps of :
f) adding a competitive agent to the sample;
g) determining interaction parameters of said two analytes by fluorescence cross correlation spectroscopy;
h) comparing the interaction parameters obtained in steps e) and g);
i) determining interaction parameters for said competitive agent by said comparison,
**characterized in that** said competitive agent is a small molecule compound not being a protein, a peptide or a nucleic acid which is not fluorescently labelled; and **characterized in that** the second analyte is a fluorescent dye labelled small molecule compound, not being a protein, a peptide or a nucleic acid.

3. Method of determining interaction parameters for one competitive agent influencing the interaction of two analytes comprising at least the steps of:
a) providing a fluorescence cross correlation spectroscopy device comprising a loading zone for a sample;
b) providing a lysate of cells cultured outside the human or animal body as sample wherein said lysate comprises the first analyte in the form of a fusion protein comprised of a target protein and an autofluorescent protein;
c) adding a competitive agent to the sample of step b);
d) loading the sample obtained in step c) onto the loading zone of the fluorescence cross correlation spectroscopy device;
e) determining interaction parameters by fluorescence cross correlation spectroscopy;
f) adding the second analyte in the form of a fluorescent dye labelled small molecule compound to the sample;
g) determining interaction parameters of said two analytes by fluorescence cross correlation spectroscopy;
h) comparing the interaction parameters obtained in steps e) and g);
i) determining interaction parameters for said competitive agent by said comparison,
**characterized in that** said competitive agent is a small molecule compound not being a protein, a peptide or a nucleic acid; and
**characterized in that** the second analyte is a fluorescent dye labelled small molecule compound, not being a protein, a peptide or a nucleic acid.

4. Method according to any of the preceding claims wherein said cells cultured outside the human or animal body are mammalian cells selected from the group of cells comprising HEK 293, HEK 293 T, HeLa and HUVEC cells.

5. Method according to any of the preceding claims wherein said cells are lysed by a protocol comprising at least the steps of collecting and washing cells, adding hypotonic buffer not comprising any detergent, douncing, restoring physiological salt conditions and collecting the supernatant after an ultracentrifugation.

6. Method according to any of the preceding claims wherein said autofluorescent protein is selected from the group of autofluorescent proteins comprising GFP, YFP, CFP and RFP.

7. Method according any of the preceding claims wherein said fluorescent dye is selected from the group of fluorescent dyes comprising Cy3, Cy5 and Alexa-dyes as well as derivatives thereof.

8. Method according to any of the preceding claims wherein the fluorescent labels are different fluorescent labels regarding their fluorescent properties when used together in a sample.

9. Method according to any of the preceding claims wherein GFP and Cy5 or RFP and Alexa-488 are used in combination as different fluorescent labels of the two analytes in a sample.

10. Method according to any of the preceding claims wherein the interaction parameters comprise affinity parameters and kinetic parameters of the reaction.

11. Method according to claim 10 wherein said affinity parameters comprise Kd-, Ki- and IC50-values.

12. Method according to claim 10 wherein said kinetic parameters comprise kon-, koff- and kobs-values.

## Patentansprüche

1. Verfahren zur Bestimmung der Interaktionsparameter von zwei Analyten, umfassend mindestens die folgenden Schritte:
a) Bereitstellen eines Fluoreszenz-Kreuzkorrelations-Spektroskopie Apparats umfassend eine Ladezone für eine Probe;
b) Bereitstellen eines Lysats von Zellen, die außerhalb des menschlichen oder tierischen Körpers gezüchtet wurden, als Probe, wobei das genannte Lysat den ersten Analyten in Form eines Fusionsproteins, umfassend ein Zielprotein und ein autofluoreszierendes Protein, umfasst;
c) Zugabe des zweiten Analyten zur Probe aus Schritt b);
d) Laden der Probe umfassend die zwei genannten Analyten auf die Ladezone des Fluoreszenz-Kreuzkorrelations-Spektroskopie-Apparats;
e) Bestimmen der Interaktionsparameter der zwei genannten Analyten durch Fluoreszenz-Kreuzkorrelations-Spektroskopie;
**dadurch gekennzeichnet, dass** der zweite Analyt eine mit einem fluoreszierenden Farbstoff markierte niedermolekulare Verbindung ist, und nicht ein Protein, ein Peptid oder eine Nukleinsäure.

2. Verfahren zur Bestimmung der Interaktionsparameter für ein kompetitierendes Agens, das die Interaktion von zwei Analyten beeinflusst, umfassend mindestens die Schritte a) bis e) von Anspruch 1 und weiter umfassend die folgenden Schritte:
f) Zugabe eines kompetitierenden Agens zur Probe;
g) Bestimmen der Interaktionsparameter der zwei genannten Analyten durch Fluoreszenz-Kreuzkorrelations-Spektroskopie;
h) Vergleichen der Interaktionsparameter, die in den Schritten e) und g) erhalten wurden;
i) Bestimmen der Interaktionsparameter für das genannte kompetitierende Agens durch genannten Vergleich,
**dadurch gekennzeichnet, dass** das genannte kompetitierende Agens eine niedermolekulare Verbindung ist und nicht ein Protein, ein Peptid oder eine Nukleinsäure, welche nicht fluoreszierend markiert ist; und **dadurch gekennzeichnet, dass** der zweite Analyt eine mit einem fluoreszierenden Farbstoff markierte niedermolekulare Verbindung ist, und nicht ein Protein, ein Peptid oder eine Nukleinsäure.

3. Verfahren zur Bestimmung der Interaktionsparameter für ein kompetitierendes Agens, das die Interaktion von zwei Analyten beeinflusst, umfassend mindestens die folgenden Schritte:
a) Bereitstellen eines Fluoreszenz-Kreuzkorrelations-Spektroskopie Apparats umfassend eine Ladezone für eine Probe;
b) Bereitstellen eines Lysats von Zellen, die außerhalb des menschlichen oder tierischen Körpers gezüchtet wurden, als Probe, wobei das genannte Lysat den ersten Analyten in Form eines Fusionsproteins, umfassend ein Zielprotein und ein autofluoreszierendes Protein, umfasst;
c) Zugabe eines kompetitierenden Agens zur Probe aus Schritt b);
d) Laden der Probe aus Schritt c) auf die Ladezone des Fluoreszenz-Kreuzkorrelations-Spektroskopie-Apparats;
e) Bestimmen der Interaktionsparameter durch Fluoreszenz-Kreuzkorrelations-Spektroskopie;
f) Zugabe des zweiten Analyten in Form einer mit einem fluoreszierenden Farbstoff markierten niedermolekularen Verbindung zur Probe;
g) Bestimmen der Interaktionsparameter der zwei genannten Analyten durch Fluoreszenz-Kreuzkorrelations-Spektroskopie;
h) Vergleichen der Interaktionsparameter, die in den Schritten e) und g) erhalten wurden;
i) Bestimmen der Interaktionsparameter für das genannte kompetitierende Agens durch genannten Vergleich,
**dadurch gekennzeichnet, dass** das genannte kompetitierende Agens eine niedermolekulare Verbindung ist und nicht ein Protein, ein Peptid oder eine Nukleinsäure; und **dadurch gekennzeichnet, dass** der zweite Analyt eine mit einem fluoreszierenden Farbstoff markierte niedermolekulare Verbindung ist, und nicht ein Protein, ein Peptid oder eine Nukleinsäure.

4. Verfahren gemäß einem der vorhergehenden Ansprüche, wobei die genannten Zellen, die außerhalb des menschlichen oder tierischen Körpers gezüchtet wurden, Säugerzellen ausgewählt aus der Gruppe von Zellen umfassend HEK 293, HEK 293 T, HeLa und HUVEC Zellen sind.

5. Verfahren gemäß einem der vorhergehenden Ansprüche, wobei die genannten Zellen durch ein Protokoll lysiert werden umfassend mindestens die Schritte des Aufnehmens und des Waschens der Zellen, der Zugabe von hypotonischen Puffer, der kein Detergenz umfasst, des Dounce-Homogenisieren, der Wiederherstellung physiologischer Salzbedingungen und der Aufnahme des Überstands nach Ultrazentrifugation.

6. Verfahren gemäß einem der vorhergehenden Ansprüche, wobei das genannte autofluoreszierende Protein ausgewählt ist aus der Gruppe autofluoreszierender Proteine umfassend GFP, YFP, CFP und RFP.

7. Verfahren gemäß einem der vorhergehenden Ansprüche, wobei der genannte fluoreszierende Farbstoff ausgewählt ist aus der Gruppe von Fluoreszenzfarbstoffen umfassend Cy3, Cy5 und Alexa-Farbstoffen sowie Derivaten davon.

8. Verfahren gemäß einem der vorhergehenden Ansprüche, wobei die fluoreszierenden Markierungen unterschiedliche fluoreszierende Markierungen bezüglich ihrer fluoreszierenden Eigenschaften sind, wenn sie zusammen in einer Probe verwendet werden.

9. Verfahren gemäß einem der vorhergehenden Ansprüche, wobei GFP und Cy5 oder RFP und Alexa-488 in Kombination als unterschiedliche fluoreszierende Markierungen der genannten zwei Analyten in einer Probe verwendet werden.

10. Verfahren gemäß einem der vorhergehenden Ansprüche, wobei die Interaktionsparameter Affinitätsparameter und kinetische Parameter der Reaktion umfassen.

11. Verfahren gemäß Anspruch 10, wobei die genannten Affinitätsparameter Kd-, Ki- und IC50-Werte umfassen.

12. Verfahren gemäß Anspruch 10, wobei die genannten kinetische Parameter kon-, koff- und kobs-Werte umfassen.

## Revendications

1. Procédé pour déterminer les paramètres d'interaction de deux analytes, comprenant au moins les étapes :
a) de fourniture d'un dispositif de spectroscopie de corrélation croisée à fluorescence comprenant une zone de charge pour un échantillon ;
b) de fourniture d'un lysat de cellules cultivées à l'extérieur de l'organisme d'un être humain ou d'un animal comme échantillon, ledit lysat comprenant le premier analyte sous forme d'une protéine de fusion constituée d'une protéine cible et d'une protéine autofluorescente ;
c) d'addition du second analyte à l'échantillon de l'étape b) ;
d) de charge de l'échantillon comprenant lesdits deux analytes sur la zone de charge du dispositif de spectroscopie de corrélation croisée à fluorescence ;
e) de détermination des paramètres d'interaction desdits deux analytes par spectroscopie de corrélation croisée à fluorescence ;
**caractérisé en ce que** le second analyte est un composé sous forme de petite molécule marquée avec un colorant fluorescent, ne consistant pas en une protéine, un peptide ou un acide nucléique.

2. Procédé pour déterminer les paramètres d'interaction pour un agent compétitif influençant l'interaction de deux analytes, comprenant au moins au moins les étapes a) à e) de la revendication 1 et comprenant en outre les étapes :
f) d'addition d'un agent compétitif à l'échantillon ;
g) de détermination des paramètres d'interaction desdits deux analytes par spectroscopie de corrélation croisée à fluorescence ;
h) de comparaison des paramètres d'interaction obtenus dans les étapes e) et g);
i) de détermination des paramètres d'interaction pour ledit agent compétitif par ladite comparaison,
**caractérisé en ce que** ledit agent compétitif est un composé sous forme de petite molécule ne consistant pas en une protéine, un peptide ou un acide nucléique, qui n'est pas marqué par fluorescence ; et **caractérisé en ce que** le second analyte est un composé sous forme de petite molécule marquée avec un colorant fluorescent, ne consistant pas en une protéine, un peptide ou un acide nucléique.

3. Procédé pour déterminer les paramètres d'interaction pour un agent compétitif influençant l'interaction de deux analytes, comprenant au moins au moins les étapes :
a) de fourniture d'un dispositif de spectroscopie de corrélation croisée à fluorescence comprenant une zone de charge pour un échantillon ;
b) de fourniture d'un lysat de cellules cultivées à l'extérieur de l'organisme d'un être humain ou d'un animal comme échantillon, ledit lysat comprenant le premier analyte sous forme d'une protéine de fusion constituée d'une protéine cible et d'une protéine autofluorescente ;
c) d'addition d'un agent compétitif à l'échantillon de l'étape b) ;
d) de charge de l'échantillon obtenu dans l'étape c) dans la zone de charge du dispositif de spectroscopie de corrélation croisée à fluorescence ;
e) de détermination des paramètres d'interaction par spectroscopie de corrélation croisée à fluorescence ;
f) d'addition du second analyte sous forme d'un composé sous forme de petite molécule marquée avec un colorant fluorescent à l'échantillon ;
g) de détermination des paramètres d'interaction desdits deux analytes par spectroscopie de corrélation croisée à fluorescence ;
h) de comparaison des paramètres d'interaction obtenus dans les étapes e) et g);
i) de détermination des paramètres d'interaction pour ledit agent compétitif par ladite comparaison,
**caractérisé en ce que** ledit agent compétitif est un composé sous forme de petite molécule ne consistant pas en une protéine, un peptide ou un acide nucléique ; et **caractérisé en ce que** le second analyte est un composé sous forme de petite molécule marquée avec un colorant fluorescent, ne consistant pas en une protéine, un peptide ou un acide nucléique.

4. Procédé suivant l'une quelconque des revendications précédentes, dans lequel lesdites cellules cultivées à l'extérieur de l'organisme d'un être humain ou d'un animal sont des cellules de mammifère choisies dans le groupe de cellules comprenant des cellules HEK 293, HEK 293 T, HeLa et HUVEC.

5. Procédé suivant l'une quelconque des revendications précédentes, dans lequel lesdites cellules sont lysées par un protocole comprenant au moins les étapes de collecte et de lavage de cellules, d'addition d'un tampon hypotonique ne comprenant aucun détergent, d'homogénéisation dans un homogénéisateur Dounce, de rétablissement de conditions de sels physiologiques et de collecte du surnageant après une ultracentrifugation.

6. Procédé suivant l'une quelconque des revendications précédentes, dans lequel ladite protéine autofluorescente est choisie dans le groupe de protéines autofluorescentes comprenant GFP, YFP, CFP et RFP.

7. Procédé suivant l'une quelconque des revendications précédentes, dans lequel ledit colorant fluorescent est choisi dans le groupe de colorants fluorescents comprenant Cy3, Cy5 et les colorants Alexa, ainsi que leurs dérivés.

8. Procédé suivant l'une quelconque des revendications précédentes, dans lequel les marqueurs fluorescents sont différents marqueurs fluorescents en rapport avec leurs propriétés fluorescentes lors de leur utilisation ensemble dans un échantillon.

9. Procédé suivant l'une quelconque des revendications précédentes, dans lequel GFP et Cy5 ou RFP et Alexa-488 sont utilisés en association comme marqueurs fluorescents différents des deux analytes dans un échantillon.

10. Procédé suivant l'une quelconque des revendications précédentes, dans lequel les paramètres d'interaction comprennent des paramètres d'affinité et des paramètres cinétiques de la réaction.

11. Procédé suivant la revendication 10, dans lequel lesdits paramètres d'affinité comprennent les valeurs de Kd, de Ki et de CI₅₀.

12. Procédé suivant la revendication 10, dans lequel lesdits paramètres cinétiques comprennent les valeurs de kₒₙ, de k_{off} et de k_{ob}.
